# FASCICULE DE BREVET EUROPEEN

(11) **EP 1 353 642 B1**
(45) Date de publication et mention de la délivrance du brevet: **07.05.2008**
(21) Numéro de dépôt: 02711921.3
(22) Date de dépôt: 04.01.2002
(51) Int. Cl.: A61K 8/81, A61Q 17/04

(54) **COMPOSITIONS PHOTOPROTECTRICES COMPRENANT UN COPOLYMERE AMPHIPHILE CONSTITUE DE MOTIFS ACIDE 2-ACRYLAMIDO-2-PROPANESULFONIQUE ET (METH)ACRYLATES**
KOSMETISCHE LICHTSCHUTZZUBEREITUNGEN ENTHALTEND EIN AMPHIPHILES COPOLYMER AUS 2-ACRYLAMIDO-2-METHYLPROPANESULFONSÄURE UND (METH)ACRYLAT
PHOTOPROTECTIVE COMPOSITIONS COMPRISING AN AMPHIPHILIC COPOYLMER COMPRISING 2-ACRYLAMIDO-2-PROPANESULFONIC AND (METH)ACRYLATES

(30) Priorité: 11.01.2001 FR 0100387
(43) Date de publication de la demande: 22.10.2003
(73) Titulaire: L'ORÉAL, 75008 Paris (FR)
(72) Inventeur: BOUTELET, Karl, F-75011 Paris (FR); CANDAU, Didier, F-91570 Bièvres (FR)
(74) Mandataire: Miszputen, Laurent
(86) Numéro de dépôt international: PCT/FR2002/000028
(87) Numéro de publication internationale: WO 2002/055045

(56) Documents cités:
- EP-A- 0 406 042
- EP-A- 0 750 899
- EP-A- 0 815 843
- EP-A- 1 055 406
- WO-A-00/31154
- US-A- 4 460 732
- US-A- 4 861 499
- US-A- 5 089 578
- US-A- 5 114 706
- US-A- 5 464 452
- US-A- 5 869 030
- US-A- 5 968 481
- A KOBAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)

## Description

La présente invention a pour objet une composition cosmétique ou dermatologique, comportant au moins un système photoprotecteur capable de filtrer les rayons UV contenant au moins un filtre UV, minéral ou organique, insoluble de taille de particule allant de 5 nm à 5 µm, caractérisée par le fait qu'elle comprend en plus au moins un polymère amphiphile particulier comportant au moins un monomère à insaturation éthylènique à groupement sulfonique, sous forme libre ou partiellement ou totalement neutralisée et comprenant au moins une partie hydrophobe.

L'invention concerne également leur application à la protection de la peau et des cheveux contre les effets du rayonnement ultraviolet.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques, lipophiles et/ou des nanopigments minéraux d'oxydes métalliques, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras ; cependant, elles perdent également plus facilement leur efficacité en protection UV dès lors qu'elles viennent en contact avec l'eau ; en effet, les filtres hydrophiles, ont tendance à disparaître à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

On peut disposer de compositions antisolaires présentant une résistance à l'eau améliorée en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

Ainsi, le besoin subsiste toujours quant à pouvoir disposer de compositions antisolaires apportant à la peau et/ou aux cheveux une protection solaire efficace, stable dans le temps et résistante à l'eau (rémanence à l'eau) et dont les performances cosmétiques seraient comparables à celles obtenues avec les émulsions huile/eau classiques.

Les filtres UV les plus couramment utilisés sont organiques et solubles dans les huiles ou dans les milieux aqueux; ils possèdent généralement dans leur structure un groupe chromophore relié à un groupe solubilisant qui est généralement une chaîne grasse dans le cas des filtres UV liposolubles ou bien un groupe acide carboxylique ou sulfonique dans le cas des filtres UV hydrosolubles.

On connaît dans l'art antérieur des filtres UV organiques insolubles micronisés de taille moyenne de particule allant de 10 nm à 2 µm qui présentent l'avantage d'être plus efficaces que leurs homologues solubles comportant le même groupe chromophore à un taux équivalent. Ce type de filtres UV est notamment décrit dans les demandes de brevet EP746 305 et EP8405395.

On utilise très fréquemment dans les formulations antisolaires en association avec les filtres UV organiques solubles des pigments ou des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium. Ce type de filtre UV insoluble permet d'augmenter le niveau de protection des filtres UV organiques solubles et d'atteindre des facteurs de protection élevés.

Malheureusement, l'incorporation des filtres UV insolubles dans les formulations solaires classiques telles que des émulsions huile/eau ou eau/huile est souvent difficile à mettre en oeuvre.

La Demanderesse a découvert de manière surprenante et inattendue que des compositions particulières contenant au moins un filtre UV insoluble et au moins un polymère amphiphile particulier comportant au moins un monomère à insaturation éthylènique à groupement sulfonique, sous forme libre ou partiellement ou totalement neutralisée et comprenant au moins une partie hydrophobe permettaient non seulement d'obtenir des compositions antisolaires stables dont les performances cosmétiques étaient comparables à celles obtenues généralement avec une composition antisolaire classique sous forme d'émulsion huile/eau mais aussi présentaient une rémanence à l'eau améliorée.

Ces découvertes sont à l'origine de la présente invention.

La présente invention a pour objet une composition cosmétique ou dermatologique, comportant au moins un filtre UV insoluble, minéral ou organique, ayant une taille moyenne des particules varie de 5 nanomètres à 5 µm, caractérisée par le fait qu'elle comprend en plus au moins un polymère amphiphile comportant au moins un monomère à insaturation éthylènique à groupement sulfonique, sous forme libre ou partiellement ou totalement neutralisée et comprenant au moins une partie hydrophobe choisi parmi les copolymères constitués de motifs acide 2-acrylamido-2-méthylpropane-sulfonique (AMPS) de formule (II) suivante : 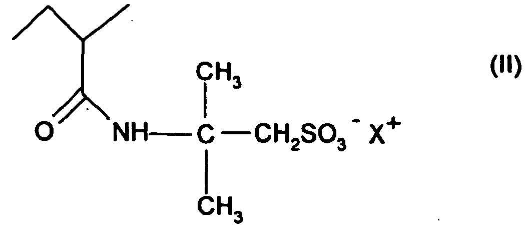 dans laquelle X⁺ est un proton, un cation de métal alcalin, un cation alcalino-terreux ou l'ion ammonium,
et de motifs de formule (III) suivante : 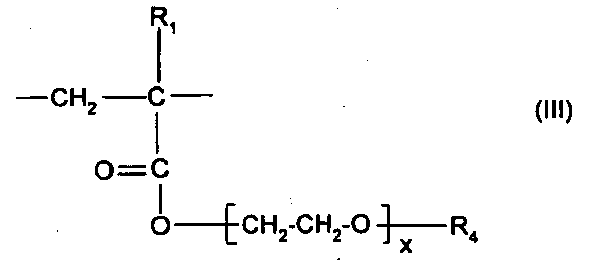 dans laquelle x désigne un nombre entier variant de 3 à 100, de préférence de 5 à 80 et plus préférentiellement de 7 à 25 ; R₁ désigne un atome d'hydrogène ou un radical alkyle linéaire ou ramifié en C₁-C₆ et R₄ désigne un alkyle linéaire ou ramifié en C₆-C₂₂ et plus préférentiellement en C₁₀-C₂₂.

Par filtre UV insoluble, au sens de la présente invention, on entend par tout filtre UV organique ou minéral ayant une solubilité dans l'eau inférieure à 0,1 % en poids et une solubilité inférieure à 1 % en poids dans la plupart des suivants organiques comme l'huile de paraffine, les benzoates d'alcools gras et les triglycérides d'acides gras, par exemple le Miglyol® 812 commercialisé par la société DYNAMIT NOBEL. Cette solubilité, définie à 70 °C comme la quantité de produit en solution dans le solvant à l'équilibre avec un excès de solide en suspension, peut facilement être évaluée au laboratoire.

La présente invention a également pour objet l'utilisation de l'émulsion pour la fabrication de compositions cosmétiques pour la protection de la peau et/ou des cheveux contre le rayonnement ultraviolet, en particulier le rayonnement solaire.

D'autres caractéristiques, aspects et avantages de la présente invention apparaîtront à la lecture de la description détaillée qui va suivre.

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe.

On entend par polymère amphiphile, tout polymère comportant à la fois une partie hydrophile et une partie hydrophobe et notamment une chaîne grasse.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non- réticulés.
De préférence, on choisit des polymères amphiphiles réticulés.
Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères à insaturation éthylénique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécylacrylamido-méthane-sulfonique ainsi que leurs formes partiellement ou totalement neutralisées.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 »;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

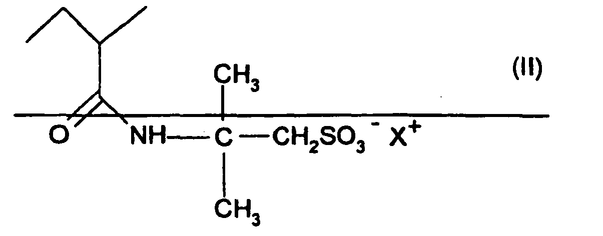

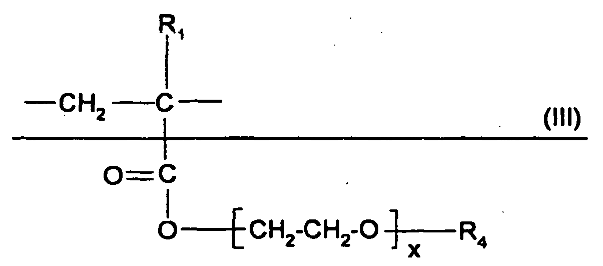

Les polymères particulièrement préférés sont ceux pour lesquels x = 25, R₁ désigne méthyle et R₄ représente n-dodécyle ; ils sont décrits dans les articles de Morishima mentionnés ci-dessus.

Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.
La distribution de la taille des particules du polymère peut être déterminée par exemple par diffraction laser ou analyse d'image.
Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.
La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.
Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
-d'un alcool en C₁₀-C₁₈ oxyéthyléné par 8 moles d'oxyde d'éthylène (GENAPOL^{®} C-080 de la société HOECHST/CLARIANT),
-d'un alcool oxo en C₁₁ oxyéthyléné par 8 moles d'oxyde d'éthylène (GENAPOL^{®} UD-080 de la société HOECHST/CLARIANT),
-d'un alcool oxo en C₁₁ oxyéthyléné par 7 moles d'oxyde d'éthylène (GENAPOL^{®} UD-070 de la société HOECHST/CLARIANT),
-d'un alcool en C₁₂-C₁₄ oxyéthyléné par 7 moles d'oxyde d'éthylène (GENAPOL^{®} LA-070 de la société HOECHST/CLARIANT),
d'un alcool en C₁₂-C₁₄ oxyéthyléné par 9 moles d'oxyde d'éthylène (GENAPOL^{®} LA-090 de la société HOECHST/CLARIANT),
-d'un alcool en C₁₂-C₁₄ oxyéthyléné par 11 moles d'oxyde d'éthylène (GENAPOL^{®} LA-110 de la société HOECHSTICLARIANT),
-d'un alcool en C₁₆-C₁₈ oxyéthyléné par 8 moles d'oxyde d'éthylène (GENAPOL^{®} T-080 de la société HOECHSTICLARIANT),
-d'un alcool en C₁₆-Cₗ₈ oxyéthyléné par 15 moles d'oxyde d'éthylène (GENAPOL^{®} T-150 de la société HOECHST/CLARIANT),
-d'un alcool en C₁₆-C₁₈ oxyéthyléné par 11 moles d'oxyde d'éthylène (GENAPOL^{®} T-110 de la société HOECHST/CLARIANT),
-d'un alcool en C₁₆-C₁₈ oxyéthyléné par 20 moles d'oxyde d'éthylène (GENAPOL^{®} T-200 de la société HOECHST/CLARIANT),
-d'un alcool en C₁₆-C₁₈ oxyéthyléné par 25 moles d'oxyde d'éthylène (GENAPOL^{®} T-250 de la société HOECHSTICLARIANT),
-d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.
De préférence pour les polymères les plus hydrophobes, la proportion molaire en motifs de formule (III) varie de 50,1 à 99,9%, plus particulièrement de 70 à 95% et encore plus particulièrement de 80 à 90%.
De préférence pour les polymères peu hydrophobes, la proportion molaire en motifs de formule (III) varie de 0,1 à 50%, plus particulièrement de 5 à 25% et encore plus particulièrement de 10 à 20%.
La distribution des monomères dans les polymères de l'invention peut être, par exemple, alternée, bloc (y compris multibloc) ou quelconque.
Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.
Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.
Ces polymères, de préférence, conduisent dans l'eau à un phénomène de démixion par chauffage se traduisant par des courbes présentant, en fonction de la température et de la concentration, un minimum appelé LCST (Lower Critical Solution Température).

Les viscosités (mesurées à 25°C au viscosimètre Brookfield aiguille 7) des solutions aqueuses à 1 % vont de préférence de 20 000 mPa.s à 100 000 mPa.s et plus particulièrement de 60 000 mPa.s à 70 000 mPa.s.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

Les filtres UV insolubles selon l'invention ont une taille moyenne des particules qui varie de 5 nanomètres (nm) à 5µm et plus préférentiellement de 10 nm à 2 µm et plus particulièrement de 20 nm à 2 µm.

Les filtres UV insolubles minéraux conformes à l'invention sont en général des pigments ou plus particulièrement des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent en particulier être obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure CₙH₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C₁-C₁₂. d'un composé de structure CₙH₂ₙ₊₁ O(C₆H₁₀O₅)ₓH et plus précisément un ester obtenu par réaction d'un acide carboxylique en C₁-C₁₂ tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre insoluble dans sa forme micronisée.

Les filtres UV organiques insolubles conformes à l'invention peuvent être choisis notamment parmi les filtres UV organiques du type oxanilide, du type triazine, du type triazole, du type amide vinylique, du type cinnamide, du type comportant un ou plusieurs groupements benzazole et/ou benzofuranne, benzothiophènene ou du type indole.

Au sens où on l'utilise dans la présente invention, le terme benzazole englobe à la fois les benzothiazoles, benzoxazoles et benzimidazoles.

Parmi les filtres UV du type oxanilide conformes à l'invention, on peut citer ceux répondant à la structure : 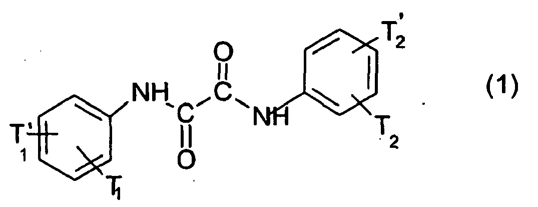 dans laquelle T₁, T'₁, T₂ et T'₂ désignent, identiques et différents, un radical alkyle en C₁-C₈ ou un radical alcoxy en C₁-C₈. Ces composés sont décrits dans la demande de brevet WO95/22959.

A titre d'exemples, on peut citer les produits commerciaux TINUVIN 315 et TINUVIN 312 vendus par la Société CIBA-GEIGY et respectivement de structure :

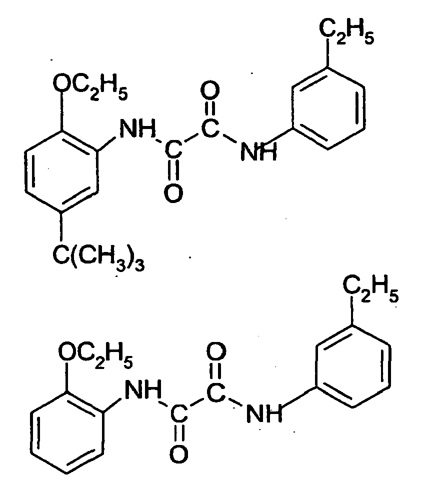

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243 (faisant partie intégrante du contenu de la description).

Parmi ces filtres UV insolubles du type triazine, on citera plus particulièrement les composés suivants :
- la 2,4,6-tris(4'-amino benzalmalonate de diéthyle)-s-triazine,
- la 2,4,6-tris(4'-amino benzalmalonate de düsopropyle)-s-triazine,
- la 2,4,6-tris(4'-amino benzalmalonate de diméthyle)-s-triazine,
- la 2,4,6-tris(a-cyano-4-aminocinnamate d'éthyle)-s-triazine.

Parmi les filtres UV insolubles du type triazine conformes à l'invention, on peut également mentionner ceux répondant à la formule (2) suivante : 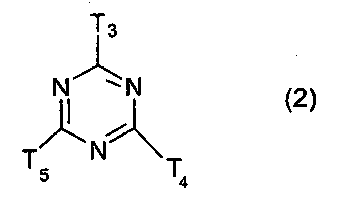 dans laquelle T₃, T₄, T₅, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OT₆, avec T₆ soit C₁-C₁₈alkyle soit cinnamyle.

Ces composés sont décrits dans WO 97/03642, GB 2286774, EP-743309, WO 98/22447, GB 2319523 (faisant partie intégrante du contenu de la description).

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroXy-5'-ter-octyl) phénylamino]-s-triazine.

Parmi les filtres UV organiques insolubles du type triazole conformes à l'invention, on peut citer ceux de formule suivante (3) tels que décrits dans la demande WO95/22959 (faisant partie intégrante du contenu de la description) : 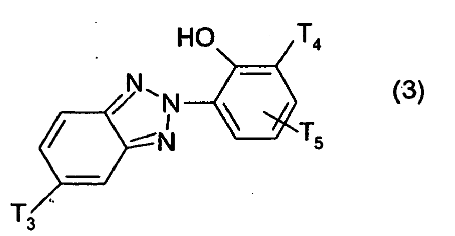 dans laquelle T₇ désigne un atome d'hydrogène ou un radical alkyle en C₁-C₁₈ ; T₈ et T₉, identiques ou différents, désignent un radical alkyle en C₁-C₁₈ éventuellement substitué par un phényle.

A titre d'exemple de composés de formule (3), on peut citer les produits commerciaux TINUVIN 328, 320, 234 et 350 de la Société CIBA-GEIGY de structure suivante :

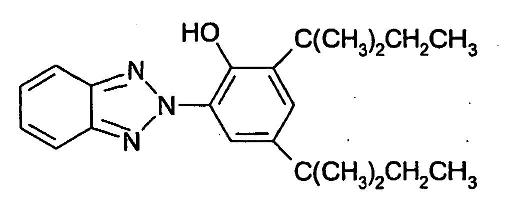

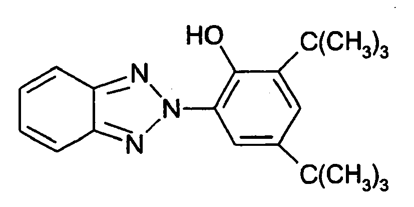

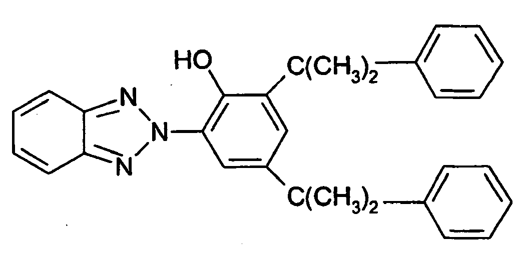

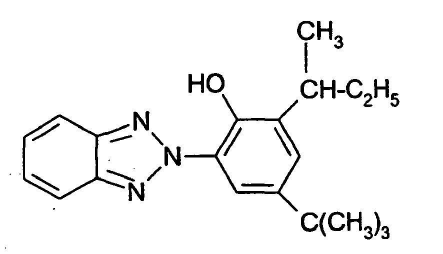

Parmi les filtres UV organiques insolubles du type triazole conformes à l'invention, on peut citer les composés tels que décrits dans les brevets US 5 687 521, US 5 687 521, US 5 373 037, US 5 362 881 et en particulier le [2,4'-dihydroxy-3-(2H-benzotriazol-2-yl)-5-(1,1,3,3-tétraméthylbutyl)-2'-n-octoxy-5'-benzoyl] diphénylméthane vendu sous le nom MIXXIM PB30 par la société FAIRMOUNT CHEMICAL de structure :

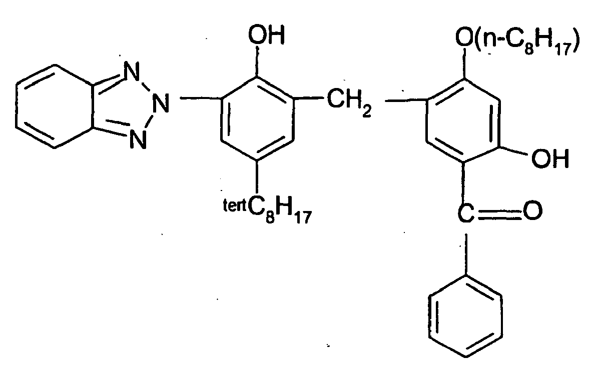

Parmi les filtres UV organiques insolubles du type benzotriazole conformes à l'invention, on peut citer les dérivés de méthylène bis-(hydroxyphényl benzotriazole) de structure suivante : 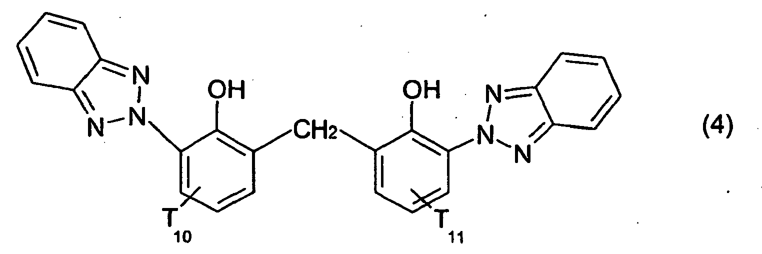 dans laquelle les radicaux T₁₀ et T₁₁ identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle. Ces composés sont connus en soi et décrits dans les demandes US 5237 071, US 5 166 355, GB-A-2 303 549, DE 197 26 184 et EP-A-893 119 (faisant partie intégrante de la description).

Dans la formule (4) définie ci-dessus : les groupes alkyle en C₁-C₁₈ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle ; les groupes cycloalkyle en C₅-C₁₂ sont par exemple cyclopentyle, cyclohexyle, cyclooctyle ; les groupes aryle sont par exemple phényle, benzyle.

Parmi les composés de formule (4), on préfère plus particulièrement ceux de structure suivante:

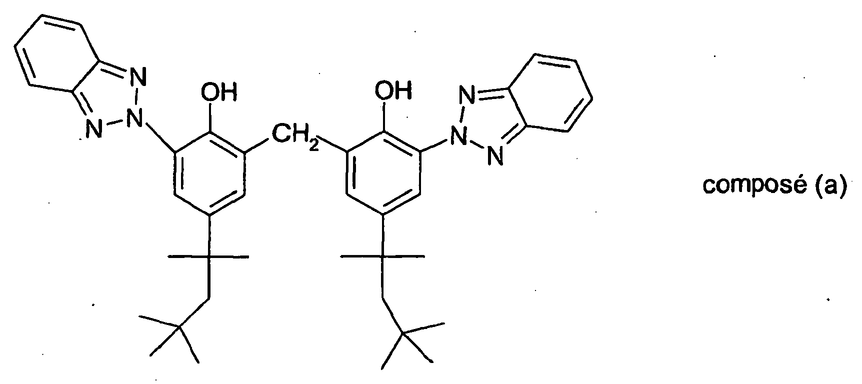

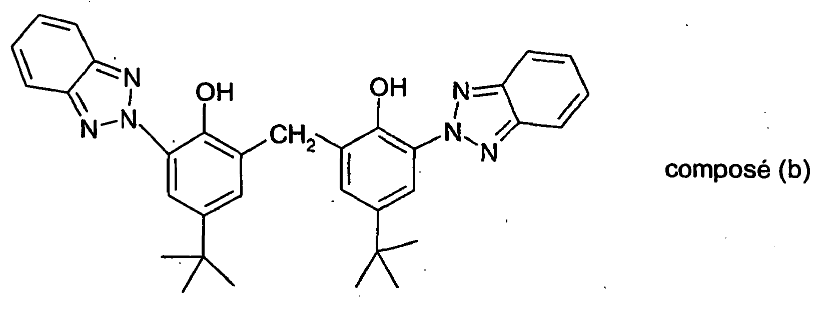

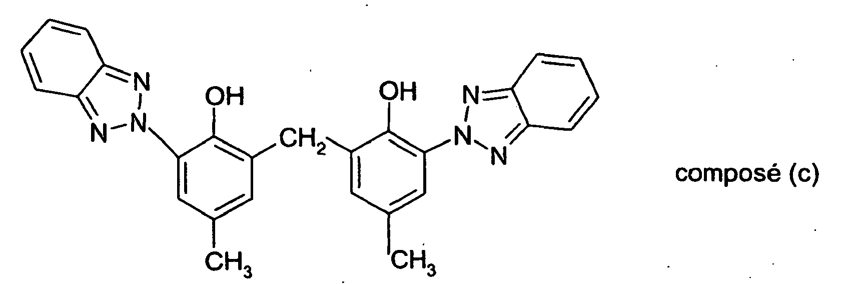

Le composé (a) de nomenclature 2,2'-méthylène-bis-[6-(2H-benzotriazol-2-yl)-4-(1,1,3,3-tetraméthylbutyl)phénol] est vendu sous forme solide le nom MIXXIM BB/100 par le société FAIRMOUNT CHEMICAL et sous forme micronisée sous le nom TINOSORB M par la société par CIBA SPECIALTY CHEMICALS.

Le composé (c) de nomenclature 2,2'-méthylène-bis-[6-(2H-benzotriazol-2-yl)-4-(methyl)phénol] est vendu sous forme solide sous le nom MIXXIM BB/200 par le société FAIRMOUNT CHEMICAL

Parmi les filtres organiques insolubles du type amide vinylique, on peut citer par exemple les composés de formules suivante qui sont décrits dans la demande WO95/22959 (faisant partie intégrante du contenu de la description) :

T₁₂-(Y)r-C(=O)-C(T₁₃)=C(T₁₄)-N(T₁₅)(T₁₆) (5)

dans laquelle T₁₂ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₇ où T₁₇ est un alkyle en C₁-C₁₈; T₁₃, T₁₄, T₁₅ et T₁₆ identiques ou différents désignent un radical alkyle en C₁-C₁₈, de préférence en C₁-C₅ ou un atome d'hydrogène ; Y est N ou O et r vaut 0 ou 1.

Parmi ces composés, on citera plus particulièrement:
- la 4-octylamino-3-pentèn-2-one ;
- l'éthyl-3-octylamino-2-buténoate ;
- la 3-octylamino-1-phényl-2-butèn-1-one,
- la 3-dodecylamino-.1-phenyl-2-buten-1-one.

Parmi les filtres organiques insolubles du type cinnamamide conformes à l'invention, on peut citer également les composés tels que décrits dans la demande WO95/22959 (faisant partie intégrante du contenu de la description) et répondant à la structure suivante : 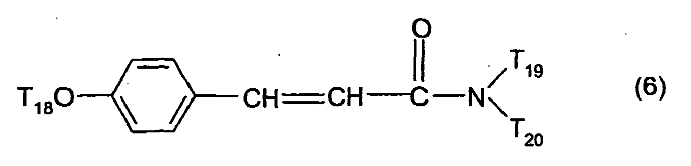 dans laquelle OT₁₈ est un radical hydroxy ou alcoxy en C₁-C₄, de préférence méthoxy ou éthoxy ; T₁₉ est hydrogène, alkyle en C₁-C₄, de préférence méthyle ou éthyle ; T₂₀ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₁ où T₂₁ est un alkyle en C₁-C₁₈ et plus préférentiellement T₂₁ est un groupe phényle, 4-méthoxyphényle ou phénylaminocarbonyle.

On peut également citer les dimères cinnamamides tels que ceux décrits dans le brevet US 5888481 comme par exemple le composé de structure :

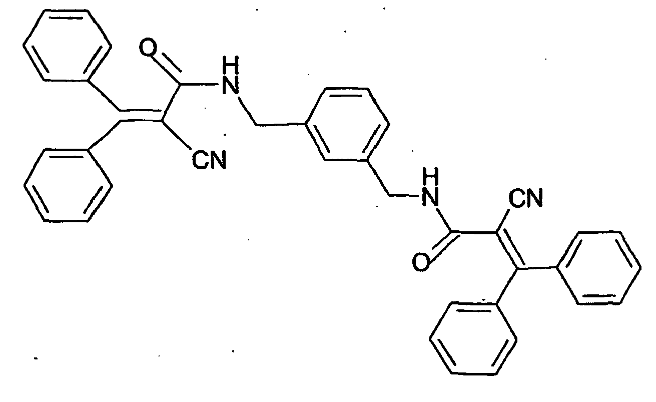

Parmi les filtres organiques insolubles du type benzazole, on peut citer ceux répondant à l'une des formules suivantes : 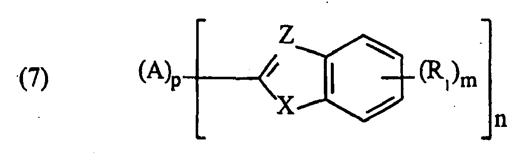 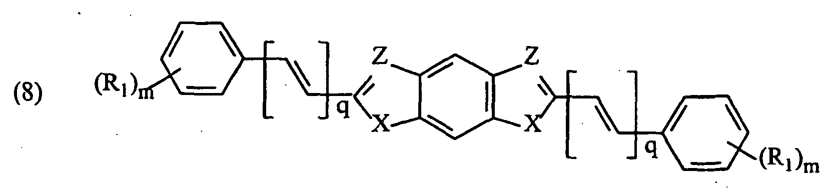 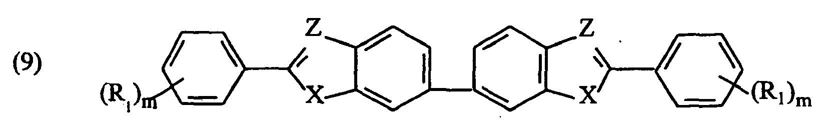 dans lesquelles chacun des symboles X représente indépendamment un atome d'oxygène ou de soufre ou un groupe NR₂,
chacun des symboles Z représente indépendamment un atome d'azote ou un groupe CH,
chacun des symboles R₁ représente indépendamment un groupe OH, un atome d'halogène, un groupe alkyle en C₁₋₈, linéaire ou ramifié, contenant éventuellement un atome de silicium, ou un groupe alcoxy en C₁₋₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₂ représente indépendamment un atome d'hydrogène, un groupe benzyle ou alkyle en C₁₋₈, linéaire ou ramifié, contenant éventuellement un atome de silicium,
A représente un radical de valence n choisi parmi ceux de formules : 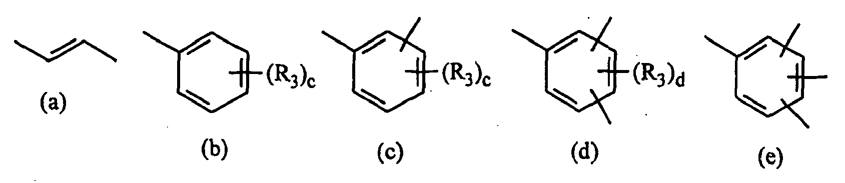 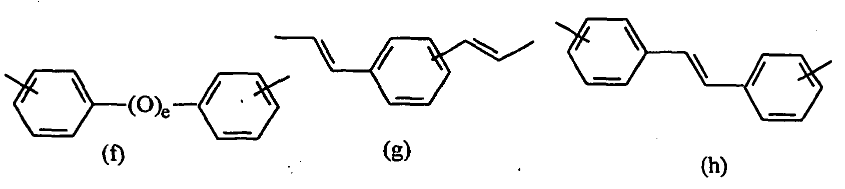 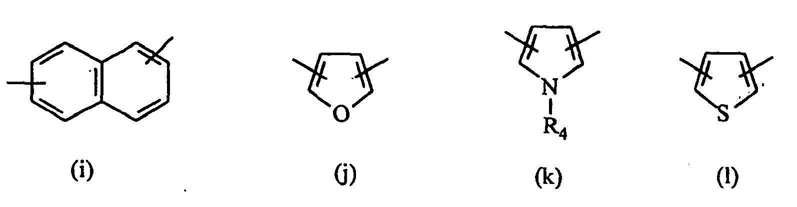 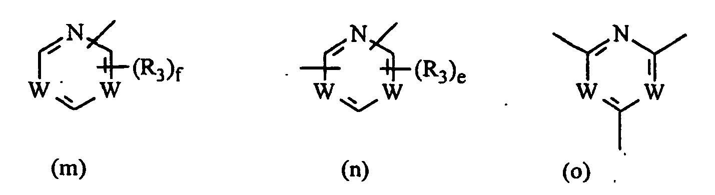 dans lesquelles chacun des symboles R₃ représente indépendamment un atome d'halogène ou un groupe alkyle ou alcoxy en C₁₋₄, linéaire ou ramifié, ou hydroxy, R₄ représente un atome d'hydrogène ou un groupe alkyle en C₁₋₄, linéaire ou ramifié,c= 0 -4, d = 0 -3, e = 0 ou 1, et f = 0 -2.

Ces composés sont notamment décrits dans les brevets DE 676 103 et CH 350 763, le brevet US 5 501 850, le brevet US 5 961 960, la demande de brevet EP0669323, le brevet US 5518713, le brevet US 2 463 264, l'article du J. Am. Chem. Soc., 79, 5706 - 5708, 1957, l'article du J. Am. Chem. Soc., 82, 609 - 611, 1960, la demande de brevet EP0921126, la demande de brevet EP712855.

A titre d'exemples de composés préférés de formule (7) de la famille des 2-arylbenzazoles, on peut mentionner le 2-benzoxazol-2-yl-4-méthylphénol, le 2-(1H-benzimidazol-2-yl)-4-méthoxyphénol ou le 2-benzothiazol-2-ylphénol, ces composés pouvant être préparés par exemple selon les procédés décrits dans le brevet CH 350 763.

A titre d'exemples de composés préférés de formule (7) de la famille des benzimidazolylbenzazoles, on citera le 2,2'-bis-benzimidazole, le 5,5',6,6'-tétraméthyl-2,2'-bis-benzimidazole, le 5,5'-diméthyl-2,2'-bis-bénzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, ces composés pouvant être préparés selon les modes opératoires décrits dans les brevets US 5 961 960 et US 2 463 264.

A titre d'exemples de composés préférés de formule (7) de la famille des phénylène-benzazoles, on citera le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(N-triméthylsilylméthyl-2-benzimidazolyle), ces composés pouvant être préparés selon les modes opératoires décrits dans le brevet US 2 463 264 et dans les publications J. Am.Chem. Soc., 82, 609 (1960) et J. Am. Chem. Soc., 79, 5706 - 5708 (1957).

A titre d'exemples de composés préférés de formule (7) de la famille des benzofuranyl-benzoxazoles, on citera le 2-(2-benzofuranyl)-benzoxazole, le 2-(benzofuranyl)-5-méthylbenzoxazole et le 2-(3-méthyl-2-benzofuranyle)-benzoxazole, ces composés pouvant être préparés selon les modes opératoires décrits dans le brevet US 5 518 713.

Comme composés préférés de formule (8), on peut citer par exemple le 2,6-diphényl-1,7-dihydro-benzo[1,2-d;4,5-d']-di-imidazole correspondant à la formule 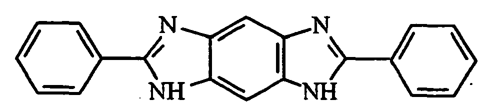 ou le 2,6-distyryl-1,7-dihydro-benzo[1,2-d ; 4,5-d']-di-imidazole ou encore le 2,6-di(p-tert-butylstyryl)-1,7-dihydrobenzo[1,2-d ; 4,5-d']-di-imidazole, qui peuvent être préparés selon la demande EP 0 669 323.

Comme composé préféré de formule (9), on peut citer le 5,5'-bis-[(phényl-2)-benzimidazole] de formule : 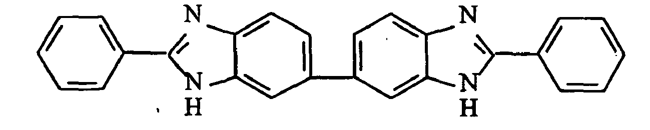 dont la préparation est décrite dans J. Chim. Phys., 64, 1602 (1967).

Parmi ces composés organiques insolubles filtrant le rayonnement UV, on préfère tout particulièrement le 2-(1H-benzimidazol-2-yl)benzoxazole, le 6-méthoxy-2,2'-bis-benzimidazole, le 2-(1H-benzimidazol-2-yl)-benzothiazole, le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(2-benzimidazolyle) et le 1,4-phénylène-bis-(N-triméthylsilylméthyl-2-benzimidazolyle).

Une autre famille particulière de filtres organiques insolubles conformes à l'invention sont les sels de métaux polyvalents (par exemple Ca²⁺, Zn²⁺, Mg²⁺, Ba²⁺, Al³⁺ ou Zr⁴⁺) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893119; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres UV insolubles de l'invention sont présents à une concentration totale comprise entre 0,1 et 25 % en poids environ, et de préférence entre 0,2 et 20 % en poids environ, par rapport au poids total de la composition.

Les compositions conformes à l'invention peuvent contenir en plus des filtres UV organiques solubles actifs dans l'UV-A et/ou l'UV-B. Ils sont choisis notamment parmi les anthranilates; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264 ; les dérivés de l'acide p-aminobenzoïque (PABA) ; les polymères filtres et silicones filtres tels que ceux décrits notamment dans la demande WO93/04665 ; les dimères dérivés d'a-alkylstyrène tels que ceux décrits dans la demande de brevet DE19855649 ; les 4,4-diarylbutadiènes tels que décrits dans les demandes de brevet EP0967200 et DE19755649 ainsi que leurs mélanges.

Comme exemples de filtres organiques complémentaires actifs dans l'UV-A et/ou l'UV-B, on peut citer désignés ci-dessus sous leur nom INCl:

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF,
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5,
- Benzophenone-6 vendu sous le nom commercial « HELISORB 11» par NORQUAY,
- Benzophenone-8 vendu sous le nom commercial « SPECTRA-SORB UV-24 » PAR AMERICAN CYANAMID,
- Benzophenone-9 vendu sous le nom commercial« UVINUL DS-49» par BASF,
- Benzophenone-12,

### Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

### Dérivés de la triazine :

- Anisotriazine vendu sous le nom commercial «TINOSORB S » par CIBA SPECIALITY CHEMICALS,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,

### Dérivés du phenyl benzotriazole :

- Drometrizole Trisiloxane vendu sous le nom « SILATRIZOLE » par RHODIA CHIMIE

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés du benzalmalonate :

- Polyorganosiloxane à fonction benzalmalonate vendu sous la dénomination commerciale « PARSOL SLX » par HOFFMANN LA ROCHE et leurs mélanges.

Les filtres UV organiques solubles plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Drometrizole Trisiloxane,
et leurs mélanges.

Le ou les filtres UV solubles complémentaires sont généralement présent dans des concentrations allant de 0,1 à 15 % en poids environ, et de préférence de 0,2 à 10 % en poids environ, par rapport au poids total de la composition.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les charges, les agents photoprotecteurs, les polymères autres que ceux de l'invention, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (HIE, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans l'utilisation d'une composition selon l'invention pour la fabrication de compositions cosmétiques pour la protection de la peau et/ou des cheveux contre le rayonnement ultraviolet, en particulier le rayonnement solaire.

Un autre objet de la présente invention réside dans l'utilisation d'un polymère amphiphile tel que défini précédemment pour la fabrication d'une composition cosmétique ou dermatologique photoprotectrice contenant au moins un filtre UV organique insoluble dans ladite émulsion, dans le but d'augmenter la résistance à l'eau de son pouvoir filtrant (rémanence à l'eau).

Des exemples concrets, mais nullement limitatifs, illustrant l'invention, vont maintenant être donnés.

### EXEMPLES DE PREPARATION:

Préparation des esters (méth)acryliques éthoxylés.

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrytate de méthyle et 20g de tétraisopropoxyde de titane. On chauffe le mélange réactionnel au reflux pendant 2 heures, et après séparation par distillation de l'alcool formé, on distille l'ester qui reste sous pression réduite. Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.
De la façon décrite ci-dessus, on a préparé les polymères suivants :
(à partir des réactants suivants en des quantités exprimées en grammes)

| Méthacrylate de Genapol T-250 | 10 | 20 | 30 | 97 |
|---|---|---|---|---|
| AMPS neutralisé par NH3 | 90 | 80 | 90 | 3 |
| Méthylène-bis-acrylamide (réticulant) | | | 1,5 | |
| Méthacrylate d'allyle (réticulant) | | 1,7 | | |
| TMPTA (réticulant) | 1,8 | | | 1,8 |
| Azobisisobutyronitrile (initiateur) | | | 1 | |
| Peroxyde dilauryle (initiateur) | 1 | 1 | | 1 |
| Tert-butanol | 300 | 300 | 300 | 300 |

| **Exemple 1 (hors invention)** | **grammes** |
|---|---|
| **PHASE A** | |
| OCTOCRYLENE | 9 |
| (Uvinul N 539 de la société BASF) | |
| BUTYL METHOXYDIBENZOYLMETHANE | 2.5 |
| (Parsol 1789 de la société Hoffmann La roche) | |
| DROMETRIZOLE TRISILOXANE | 0.75 |
| (Silatrizole de la société Rhodia) | |
| COCOATE DE DECYLE | 9 |
| (Tegosoft DC de la société Goldschmidt) | |
| **PHASE B** | |
| Copolymère acide acrylamido-2-méthyl-2-propane-sulfonique/n-dodécylacrylamide (3,5%/96,5%en poids) neutralisé à 100% par la soude | 1.5 |
| **PHASE C** | |
| GLYCEROL | 4 |
| PROPYLENE GLYCOL | 4 |
| ACIDE ETHYLENE DIAMINE TETRACETIQUE, SEL | |
| DISODIQUE | 0.1 |
| CONSERVATEURS | qs |
| TEREPHTALYLIDENE DICAMPHOR SULFONIC ACID | 1.5 |
| (Mexoryl SX de la société Chimex) | |
| TRIETHANOLAMINE | 0.26 |
| EEAU | qsp 100g |
| **PHASE D** | |
| OXYDE DE TITANE ANATASE (60 NM) ENROBE SILICE/ALUMINE EN DISPERSION AQUEUSE PROTEGEE | 16.7 |

### MODE OPERATOIRE :

On mélange les phases A et B et on chauffe à 70°C. On mélange la phase C au mélange obtenu (A+B) sous agitation du type MORITZ et on laisse refroidir. On ajoute ensuite la phase D.

### EXEMPLE 2 (invention)

Des résultats similaires ont été obtenus en remplaçant le copolymère acide acrylamido-2-méthyl-2-propane-sulfoniquein-dodécylacrylam ide neutralisé par la soude de l'exemple ci-dessus par un copolymère réticulé par du méthylène-bis-acrylamide constitué de 75% en poids de motifs AMPS neutralisés par NH₃ et de 25% en poids de motifs de formule (III) dans laquelle R₁=H, R₄=C₁₆-C₁₈ et x=25.

### EXEMPLE 3 (invention)

Des résultats similaires ont été obtenus en remplaçant le copolymère acide acrylamido-2-méthyl-2-propane-sulfoniquein-dodécylacrylamide neutralisé par la soude de l'exemple ci-dessus par un copolymère réticulé par du méthacrylate d'allyle constitué de 90% en poids de motifs AMPS neutralisés par NH₃ et de 10% en poids de motifs méthacrylate de GENAPOL T-250 [motifs de formule (III) dans laquelle R₁=CH₃, R₄=C₁₆-C₁₈ et x=25], ou par un copolymère réticulé par du méthacrylate d'allyle constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

## Revendications

1. Composition cosmétique ou dermatologique, comportant au moins un système photoprotecteur capable de filtrer les rayons UV contenant au moins un filtre UV, minéral ou organique, insoluble de taille de particule allant de 5 nm à 5 µm, **caractérisée par le fait qu'**elle comprend en plus au moins un polymère amphiphile comportant au moins un monomère à insaturation éthylènique à groupement sulfonique, sous forme libre ou partiellement ou totalement neutralisée et comprenant au moins une partie hydrophobe choisi parmi les copolymères constitués de motifs acide 2-acrylamido-2-méthylpropane-sulfonique (AMPS) de formule (II) suivante : 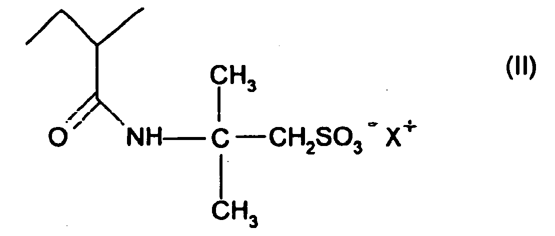 dans laquelle X⁺ est un proton, un cation de métal alcalin, un cation alcalino-terreux ou l'ion ammonium,
et de motifs de formule (III) suivante : 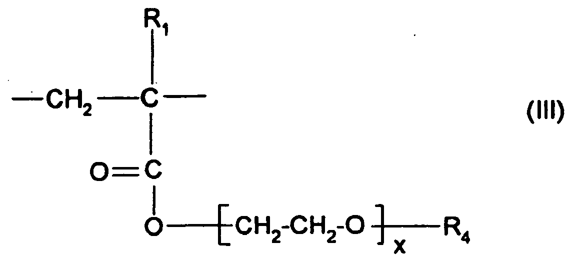 dans laquelle x désigne un nombre entier variant de 3 à 100, de préférence de 5 à 80 et plus préférentiellement de 7 à 25 ; R₁ désigne Un atome d'hydrogène ou un radical alkyle linéaire ou ramifié en C₁-C₆ et R₄ désigne un alkyle linéaire ou ramifié en C₆-C₂₂ et plus préférentiellement en C₁₀-C₂₂.

2. Composition selon la revendication 1, **caractérisée par le fait que** les polymères amphiphiles sont neutralisés partiellement ou totalement par une base minérale ou organique.

3. Composition selon la revendication 1 ou 2, **caractérisée par le fait que** les polymères amphiphiles ont un poids moléculaire moyen en nombre allant de 1000 à 20 000 000 g/mole.

4. Composition selon la revendication 3, **caractérisée par le fait que** le poids moléculaire moyen en nombre varie de 20 000 à 5 000 000 g/mole.

5. Composition selon la revendication 4, **caractérisée par le fait que** le poids moléculaire moyen en nombre varie de 100 000 à 1 500 000 g/mole.

6. Composition selon l'une quelconque des revendications précédentes, **caractérisée par le fait que** les polymères amphiphiles sont préparés par polymérisation radicalaire par précipitation dans le tert-butanol.

7. Composition selon l'une quelconque des revendications précédentes, **caractérisée par le fait que** les polymères amphiphiles sont réticulés ou non-réticulés.

8. Composition selon la revendication 7, **caractérisée par le fait que** les polymères amphiphiles sont réticulés.

9. Composition selon la revendication 8, **caractérisée par le fait que** le ou les agents de réticulation sont choisis parmi les composés à polyinsaturation oléfinique.

10. Composition selon la revendication 9, **caractérisée par le fait que** le ou les agents de réticulation sont choisis parmi le méthylène-bis-acrylamide, le méthacrylate d'allyle ou le triméthylol propane triacrylate (TMPTA).

11. Composition selon l'une quelconque des revendications 8 à 10, **caractérisée par le fait que** le taux de réticulation varie de préférence de 0,01 à 10% en moles et plus particulièrement de 0,2 à 2% en moles par rapport au polymère.

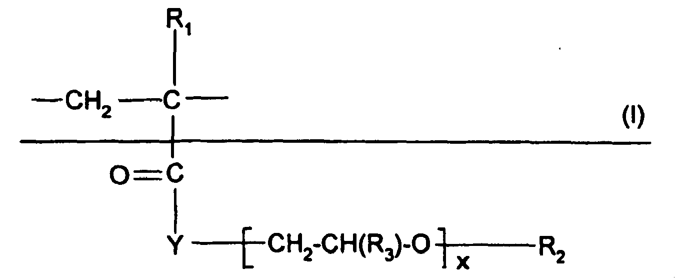

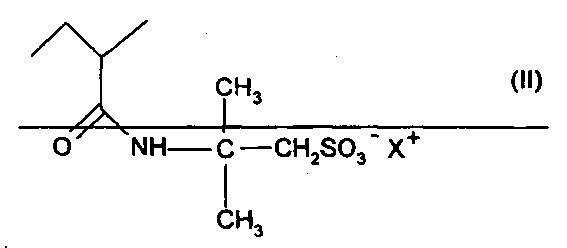

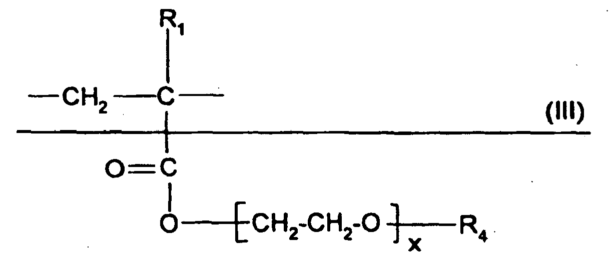

12. Composition selon l'une quelconque des revendications précédentes, **caractérisée par le fait que** x = 25, R₁ est méthyle et R₄ est n-dodécyle.

13. Composition selon l'une quelconque des revendications précédentes, **caractérisée par le fait que** la proportion molaire en % des motifs de formule (III) dans les polymères varie de 50,1 à 99,9%.

14. Composition selon l'une quelconque des revendications précédentes, **caractérisée par le fait que** la proportion molaire en % des motifs de formule (III) dans les polymères varie de 0,1 à 50%.

15. Composition selon l'une quelconque des revendications précédentes, **caractérisée par le fait que** les polymères amphiphiles sont présents dans des concentrations allant de 0,01 à 30% en poids, plus préférentiellement de 0,1 à 10% en poids, encore plus préférentiellement de 0,1 à 5% en poids, et plus particulièrement encore de 0,5 à 2% en poids par rapport au poids total de la composition.

16. Composition selon l'une quelconque des revendications 1 à 15, où le ou les filtres UV organiques insolubles sont choisis parmi les filtres UV organiques du type oxanilide, du type triazine, du type triazole, du type amide vinylique, du type cinnamide, du type benzazole.

17. Composition selon la revendication 16, où les filtres UV du type oxanilide est de formule 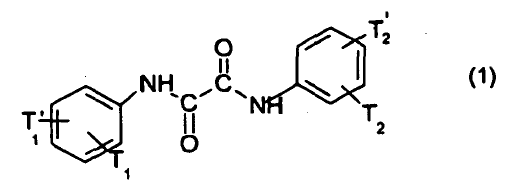 dans laquelle T₁, T'₁, T₂ et T'₂ désignent, identiques et différents, un radical alkyle en C₁-C₈ ou un radical alcoxy en C₁-C₈.

18. Composition selon la revendication 17, où les filtres UV du type oxanilide sont choisis parmi les composés suivants :

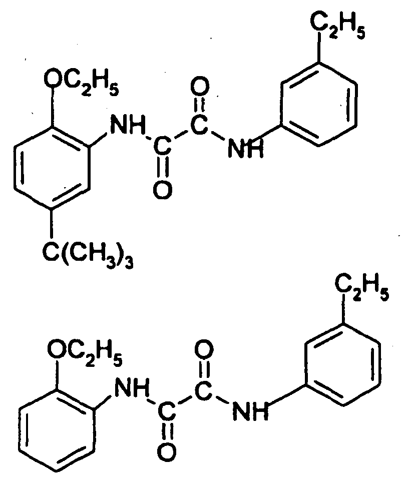

19. Composition selon la revendication 16, où les filtres UV du type triazine sont choisis parmi les dérivés insolubles de s-triazine portant des groupements benzalmalonates et/ou phénylcyanoacylates.

20. Composition selon la revendication 19, où les filtres UV du type triazine sont choisis parmi les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

21. 48. Composition selon la revendication 16, où les filtres UV insolubles du type triazine sont choisis parmi 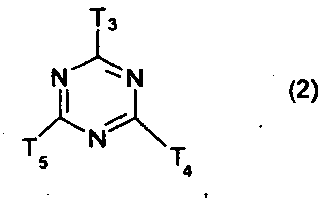 dans laquelle T₃, T₄, T₅, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyatkyte. C₅-C₈cycloalkyle, un groupe méthylidènecamphre, un groupe-(CH=CH)ₙ(CO)-OT₆. avec T₆ soit C₁-C₁₈alkyle soit cinnamyle, et n vaut 0 ou 1.

22. Composition selon la revendication 16, où les filtres UV du type triazine sont choisis parmi les dérivés insolubles de s-triazine portant des groupements benzotriazoles et/ou benzothiazoles.

23. Composition selon la revendication 22, où les filtres UV du type triazine sont choisis parmi
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

24. Composition selon la revendication 16, où les filtres UV organiques du type triazole répondent à la formule (3) suivante : 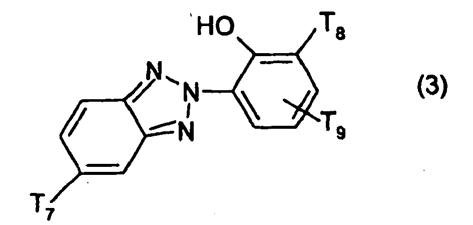 dans laquelle T₇ désigne un atome d'hydrogène ou un radical alkyle en C₁-C₁₈ ; T₈ et T₉, identiques ou différents, désignent un radical alkyle en C₁-C₁₈ éventuellement substitué par un phényle.

25. Composition selon la revendication 24, où le composé de formule (3) est choisi parmi les composés suivants :

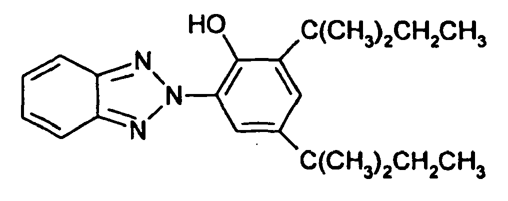

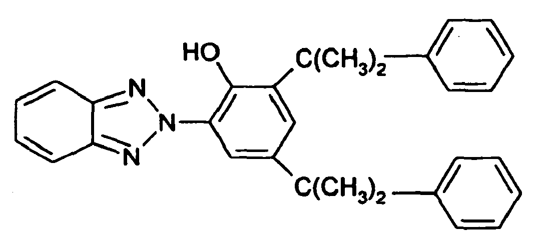

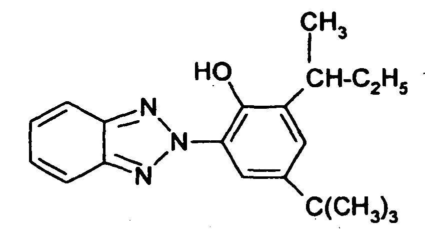

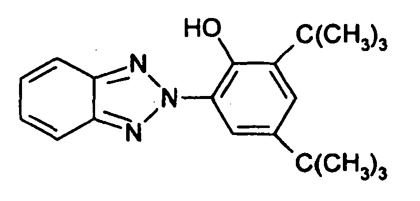

26. Composition selon la revendication 16, où le filtre UV insoluble est le [2,4'-dihydroxy-3-(2H-benzotriazol-2-yl)-5-(1,1,3,3-tétraméthylbutyl)-2'-n-octoxy-5'-benzoyl] diphénylméthane de structure :

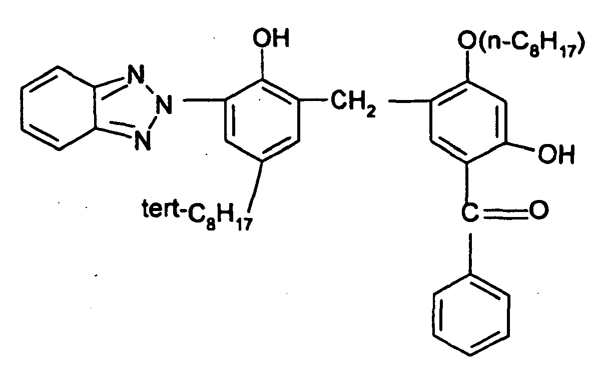

27. Composition selon la revendication 16, où les filtres UV organiques du type triazole sont choisis parmi les dérivés de méthylène bis-(hydroxyphényl benzotriazole) de structure suivante : 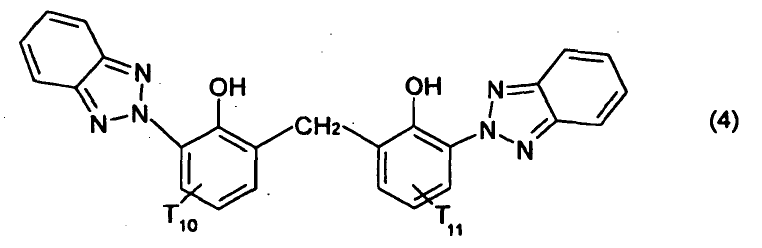 dans laquelle les radicaux T₁₀ et T₁₁ identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle.

28. Composition selon la revendication 27, où le composé de formule (4) est choisi dans le groupe constitué par les composés de structure suivante :

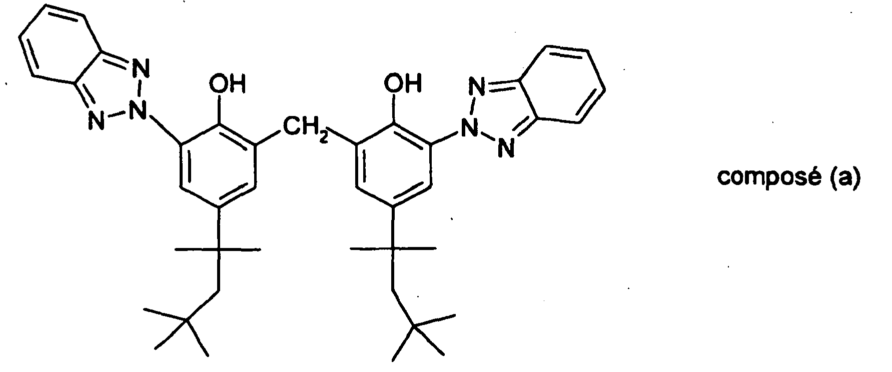

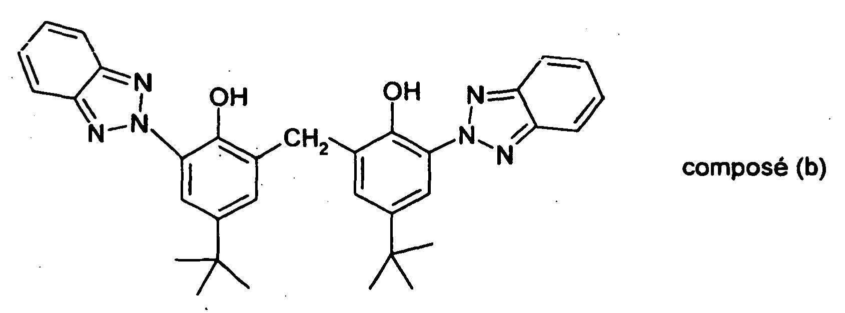

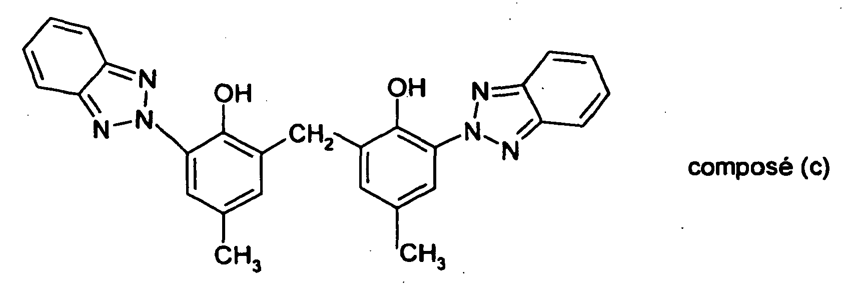

29. Composition selon la revendication 16, où les filtres organiques du type amide vinylique, répondent à la formule suivante :
T₁₂-(Y)r-C(=O)-C(T₁₃)=C(T₁₄)-N(T₁₅)(T₁₆) (5)
dans laquelle T₁₂ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₇ où T₁₇ est un alkyle en C₁-C₁₈ ; T₁₃, T₁₄, T₁₅ et T₁₆ identiques ou différents désignent un radical alkyle en C₁-C₁₈, de préférence en C₁-C₅ ou un atome d'hydrogène ; Y est N ou O et r vaut 0 ou 1.

30. Composition selon la revendication 29, où les composés de formule (5) sont choisis parmi :
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

31. Composition selon la revendication 16, où les filtres organiques du type cinnamamide répond à la formule suivante : 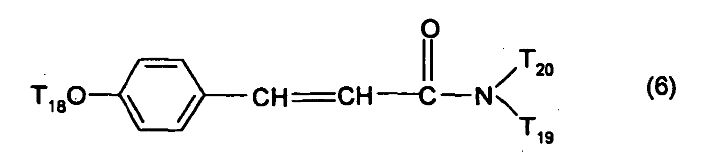 dans laquelle OT₁₈ est un radical hydroxy ou alcoxy en C₁-C₄, de préférence méthoxy ou éthoxy ; T₁₉ est hydrogène, alkyle en C₁-C₄, de préférence méthyle ou éthyle ; T₂₀ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₁ où T₂₁ est un alkyle en C₁-C₁₈.

32. Composition selon la revendication 16, où le filtre UV insoluble est un dimère cinnamamide.

33. Composition selon la revendication 32, où le filtre UV insoluble est le composé de structure :

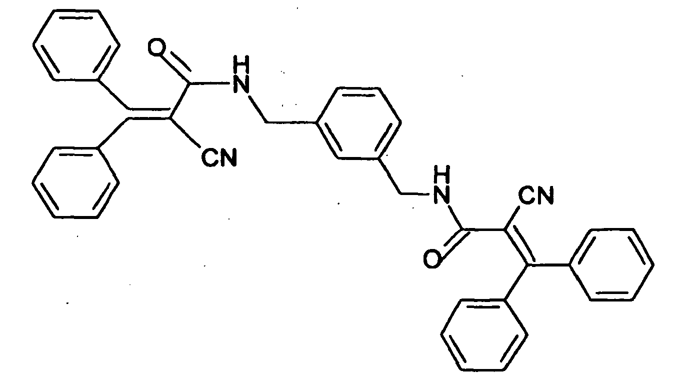

34. Composition selon la revendication 16, où les filtres UV insolubles du type benzazole sont choisis parmi ceux répondant à l'une des formules (7), (8) et (9) suivantes : 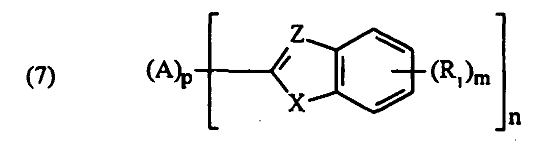 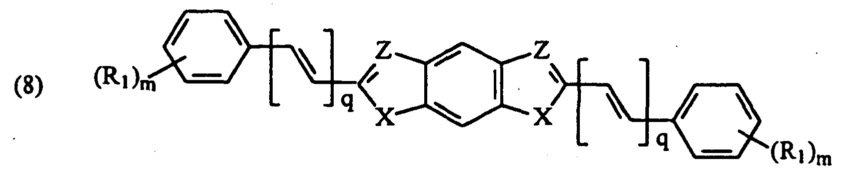 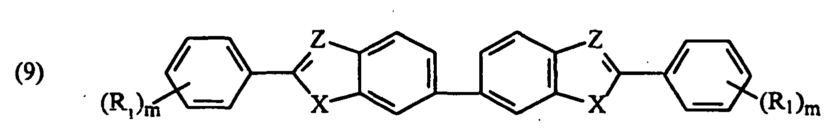 dans lesquelles
chacun des symboles X représente indépendamment un atome d'oxygène ou de soufre ou un groupe NR₂,
chacun des symboles Z représente indépendamment un atome d'azote ou un groupe CH,
chacun des symboles R₁ représente indépendamment un groupe OH, un atome d'halogène, un groupe alkyle en C₁₋₈, linéaire ou ramifié, contenant éventuellement un atome de silicium, ou un groupe alcoxy en C₁₋₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₂ représente indépendamment un atome d'hydrogène, un groupe benzyle ou alkyle en C₁₋₈, linéaire ou ramifié, contenant éventuellement un atome de silicium,
A représente un radical de valence n choisi parmi ceux de formules 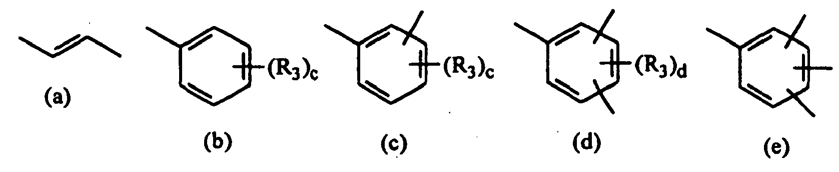 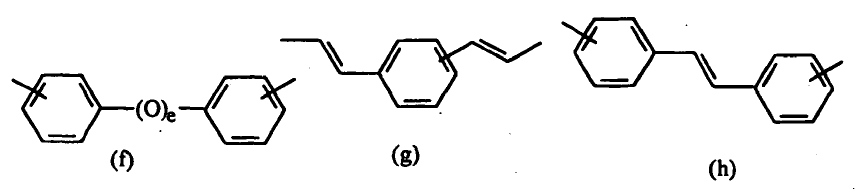 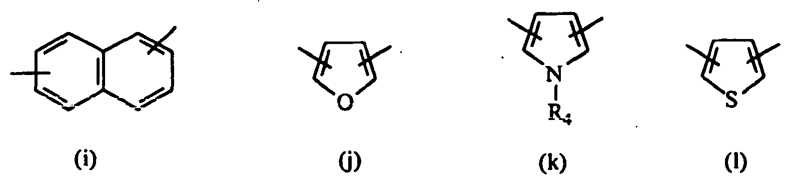 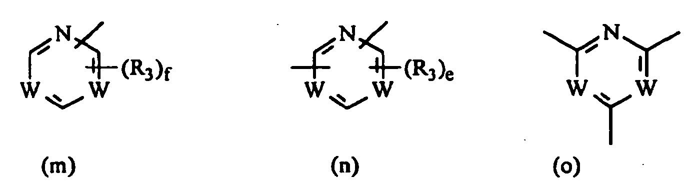 dans lesquelles
chacun des symboles R₃ représente indépendamment un atome d'halogène ou un groupe alkyle ou alcoxy en C₁₋₄, linéaire ou ramifié, ou hydroxy,
R₄ représente un atome d'hydrogène ou un groupe alkyle en C₁₋₄, linéaire ou ramifié, c=0-4,d=0-3,e=Oou 1,etf=0-2.

35. Composition selon la revendication 34, où le composé benzazole de formule (7) est choisi parmi le 2-benzoxazol-2-yl-4-méthylphénol, le 2-(1H-benzimidazol-2-yl)-4-méthoxyphénol ou le 2-benzothiazol-2-ylphénol, le 2,2'-bis-benzimidazole, le 5,5',6,6'-tétraméthyl-2,2'-bis-benzimidazole, le 5,5'-diméthyl-2,2'-bis-benzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(N-triméthylsilylméthyl-2-benzimidazolyle), le 2-(2-benzofuranyl)-benzoxazole, le 2-(benzofuranyl)-5-méthylbenzoxazole et le 2-(3-méthyl-2-benzofuranyle)-benzoxazole.

36. Composition selon la revendication 34, où le composé benzazole de formule (8) est choisi parmi le 2,6-diphényl-1,7-dihydro-benzo[1,2-d;4,5-d']-di-imidazole, le 2,6-distyryl-1,7-dihydro-benzo[1,2-d ; 4,5-d']-di-imidazole, le 2,6-di(p-tert-butylstyryl)-1,7-dihydrobenzo[1,2-d ; 4,5-d']-di-imidazole.

37. Composition selon la revendication 34, où le composé benzazole de formule (9) est le 5,5'-bis-[(phényl-2)-benzimidazole].

38. Composition selon l'une quelconque des revendications 34 et 35, où les filtres UV insolubles du type benzazole sont choisis parmi le 2-(1H-benzimidazol-2-yl)benzoxàzole, le 6-méthoxy-2,2'-bis-benzimidazole, le 2-(1H-benzimidazol-2-yl)-benzothiazole, le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(2-benzimidazolyle) et le 1,4-phénylène-bis-(N-triméthylsilylméthyl-2-benzimidazolyle).

39. Composition selon l'une quelconque des revendications 1 à 15, où les filtres UV insolubles sont des sels de métaux polyvalents de filtres UV organiques sulfoniques ou carboxyliques.

40. Composition selon la revendication 39, où les filtres UV insolubles sont choisis parmi les sels de métaux polyvalents de dérivés sulfonés de benzylidène camphre ; les sels de métaux polyvalents de dérivés sulfonés de benzimidazole; les sels de métaux polyvalents de dérivés d'acide cinnamique.

41. Composition selon l'une quelconque des revendications 1 à 15, où les filtres UV insolubles sont des complexes de métaux polyvalents ou d'ammonium ou d'ammonium substitué de filtres organiques UV-A et/ou UV-B.

42. Composition selon l'une quelconque des revendications 1 à 15, où les filtres UV insolubles minéraux sont des pigments d'oxydes métalliques enrobés ou non.

43. Composition selon la revendication 42, où les pigments d'oxydes métalliques ont une taille moyenne des particules primaires comprise entre 5 nm et 100 nm, de préférence entre 10 nm et 50 nm.

44. Composition selon la revendication 43, où les pigments d'oxydes métalliques sont des pigments d'oxyde de titane, de fer, de zinc, de zirconium ou de cérium.

45. Composition selon la revendication 44, où les pigments d'oxydes métalliques sont des pigments d'oxyde de titane amorphe ou cristallisé sous forme rutile et/ou anatase

46. Composition selon l'une quelconque des revendications 1 à 45, **caractérisée par** le fait le ou les filtres UV insolubles sont présents à une concentration totale comprise entre 0,1 et 25 % en poids environ, et de préférence entre 0,2 et 20 % en poids environ, par rapport au poids total de la composition.

47. Composition selon l'une quelconque des revendications 1 à 46, **caractérisée par le fait qu'**elle comprend en outre au moins un agent de bronzage et/ou de brunissage artificiel de la peau.

48. Composition selon l'une quelconque des revendications 1 à 47, **caractérisée par le fait qu'**elle comprend en outre au moins un filtre UV organique soluble actif dans l'UV-A et/ou l'UV-B..

49. Composition selon la revendication 48, où les filtres UV organiques solubles sont choisis notamment parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate ; les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les polymères filtres et silicones filtres ; les dimères dérivés d'α-alkylstyrène ; les 4,4-diaylbutadiènes et leurs mélanges.

50. Composition selon la revendication 49, où les filtres UV organiques solubles sont choisis parmi :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Drometrizole Trisiloxane,
et leurs mélanges.

51. Composition selon l'une quelconque des revendications 1 à 50, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les charges, les agents photoprotecteurs, les polymères autres que ceux de l'invention, les propulseurs, les agents alcalinisants ou acidifiants, les colorants.

52. Composition selon l'une quelconque des revendications 1 à 51, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

53. Composition selon l'une quelconque des revendications 1 à 52, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

54. Composition selon l'une quelconque des revendications 1 à 52, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

55. Utilisation d'un polymère amphiphile comportant au moins un monomère à insaturation éthylènique à groupement sulfonique, sous forme libre ou partiellement ou totalement neutralisée et comprenant au moins une partie hydrophobe tel que défini dans l'une quelconque des revendications 1 à 15 pour la fabrication d'une composition cosmétique ou dermatologique photoprotectrice contenant au moins un filtre organique UV insoluble, dans le but d'augmenter la résistance à l'eau de son pouvoir filtrant (rémanence à l'eau).

## Claims

1. Cosmetic or dermatological composition comprising at least one photoprotective system capable of screening out UV rays, containing at least one insoluble mineral or organic UV-screening agent, with a particle size ranging from 5 nm to 5 µm, **characterized in that** it also comprises at least one amphiphilic polymer comprising at least one ethylenically unsaturated monomer containing a sulphonic group, in free form or partially or totally neutralized form, and comprising at least one hydrophobic portion chosen from copolymers consisting of 2-acrylamido-2-methylpropanesulphonic acid (AMPS) units of formula (II) below: 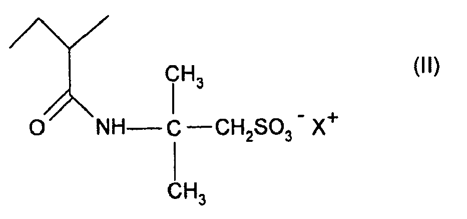 in which X⁺ is a proton, an alkali metal cation, an alkaline-earth metal cation or the ammonium ion, and of units of formula (III) below: 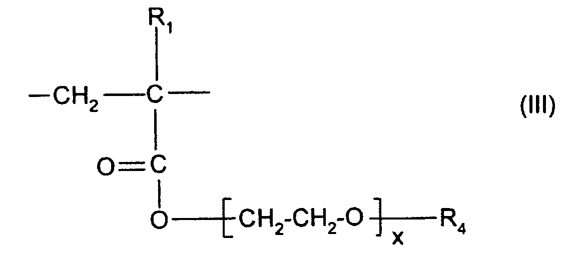 in which x denotes an integer ranging from 3 to 100, preferably from 5 to 80 and more preferably from 7 to 25; R₁ denotes a hydrogen atom or a linear or branched C₁-C₆ alkyl radical and R₄ denotes a linear or branched C₆-C₂₂ and more preferably C₁₀-C₂₂ alkyl.

2. Composition according to Claim 1, **characterized in that** the amphiphilic polymers are partially or totally neutralized with a mineral or organic base.

3. Composition according to Claim 1 or 2, **characterized in that** the amphiphilic polymers have a number-average molecular weight ranging from 1 000 to 20 000 000 g/mol.

4. Composition according to Claim 3, **characterized in that** the number-average molecular weight ranges from 20 000 to 5 000 000 g/mol.

5. Composition according to Claim 4, **characterized in that** the number-average molecular weight ranges from 100 000 to 1 500 000 g/mol.

6. Composition according to any one of the preceding claims, **characterized in that** the amphiphilic polymers are prepared by free-radical precipitation polymerization in tert-butanol.

7. Composition according to any one of the preceding claims, **characterized in that** the amphiphilic polymers are crosslinked or non-crosslinked.

8. Composition according to Claim 7, **characterized in that** the amphiphilic polymers are crosslinked.

9. Composition according to Claim 8, **characterized in that** the crosslinking agent(s) is(are) chosen from polyolefinically unsaturated compounds.

10. Composition according to Claim 9, **characterized in that** the crosslinking agent(s) is(are) chosen from methylenebisacrylamide, allyl methacrylate and trimethylolpropane triacrylate (TMPTA).

11. Composition according to any one of Claims 8 to 10, **characterized in that** the degree of crosslinking preferably ranges from 0.01 mol% to 10 mol% and more particularly from 0.2 mol% to 2 mol% relative to the polymer.

12. Composition according to any one of the preceding claims, **characterized in that** x = 25, R₁ is methyl and R₄ is n-dodecyl.

13. Composition according to any one of the preceding claims, **characterized in that** the molar percentage proportion of units of formula (III) in the polymers ranges from 50.1% to 99.9%.

14. Composition according to any one of the preceding claims, **characterized in that** the molar percentage proportion of units of formula (III) in the polymers ranges from 0.1% to 50%.

15. Composition according to any one of the preceding claims, **characterized in that** the amphiphilic polymers are present in concentrations ranging from 0.01% to 30% by weight, more preferably from 0.1% to 10% by weight, even more preferably from 0.1% to 5% by weight and even more particularly from 0.5% to 2% by weight relative to the total weight of the composition.

16. Composition according to any one of Claims 1 to 15, in which the insoluble organic UV-screening agent(s) is(are) chosen from organic UV-screening agents of the oxanilide type, of the triazine type, of the triazole type, of the vinyl amide type, of the cinnamide type or of the benzazole type.

17. Composition according to Claim 16, in which the UV-screening agents of the oxanilide type are of formula 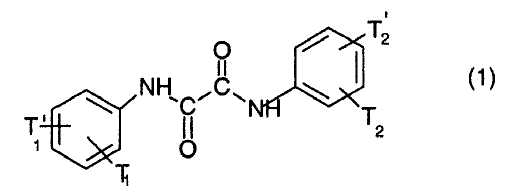 in which T₁, T'₁, T₂ and T'₂, which may be identical or different, denote a C₁-C₈ alkyl radical or a C₁-C₈ alkoxy radical.

18. Composition according to Claim 17, in which the UV-screening agents of the oxanilide type are chosen from the following compounds:

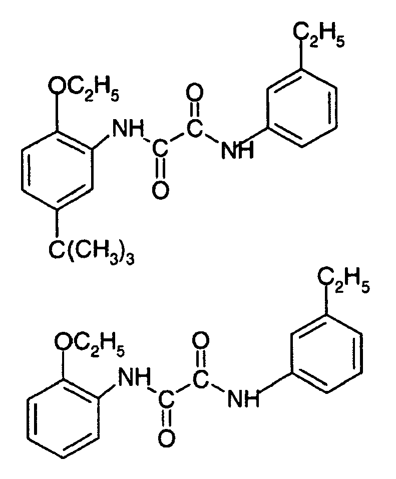

19. Composition according to Claim 16, in which the UV-screening agents of the triazine type are chosen from insoluble s-triazine derivatives bearing benzalmalonate and/or phenylcyanoacylate groups.

20. Composition according to Claim 19, in which the UV-screening agents of the triazine type are chosen from the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

21. Composition according to Claim 16, in which the insoluble UV-screening agents of the triazine type are chosen from 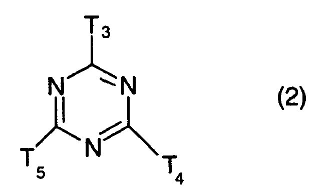 in which T₃, T₄ and T₅, independently, are phenyl, phenoxy or pyrrolo, in which the phenyl, phenoxy and pyrrolo are optionally substituted with one, two or three substituents chosen from OH, C₁-C₁₈ alkyl or alkoxy, C₁-C₁₈ carboxyalkyl, C₅-C₈ cycloalkyl, a methylidenecamphor group or a group -(CH=CH)ₙ(CO)-OT₆, with T₆ being either C₁-C₁₈ alkyl or cinnamyl, and n is 0 or 1.

22. Composition according to Claim 16, in which the UV-screening agents of the triazine type are chosen from insoluble s-triazine derivatives bearing benzotriazole and/or benzothiazole groups.

23. Composition according to Claim 22, in which the UV-screening agents of the triazine type are chosen from
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-tert-octyl)phenylamino]-s-triazine.

24. Composition according to Claim 16, in which the organic UV-screening agents of the triazole type correspond to formula (3) below: 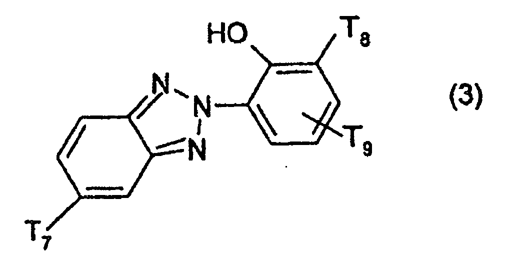 in which T₇ denotes a hydrogen atom or a C₁-C₁₈ alkyl radical; T₈ and T₉, which may be identical or different, denote a C₁-C₁₈ alkyl radical optionally substituted with a phenyl.

25. Composition according to Claim 24, in which the compound of formula (3) is chosen from the following compounds

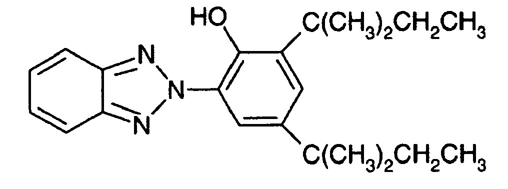

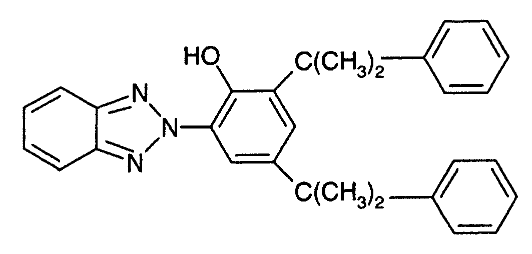

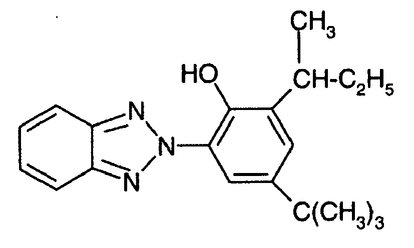

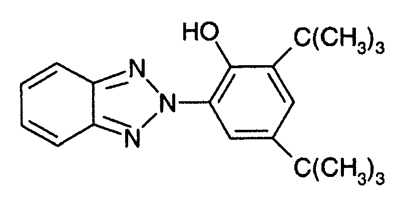

26. Composition according to Claim 16, in which the insoluble UV-screening agent is [2,4'-dihydroxy-3-(2H-benzotriazol-2-yl)-5-(1,1,3,3-tetramethylbutyl)-2'-n-octoxy-5'-benzoyl]diphenylmethane of structure:

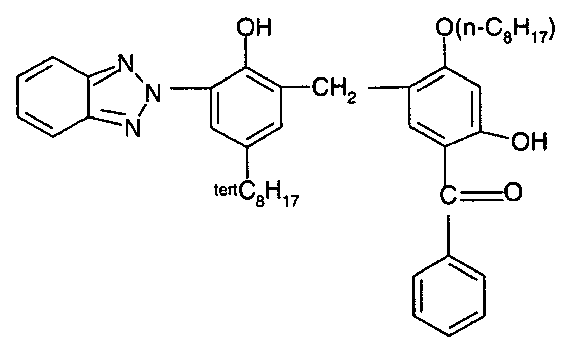

27. Composition according to Claim 16, in which the organic UV-screening agents of the triazole type are chosen from the methylenebis(hydroxyphenylbenzotriazole) derivatives of the following structure: 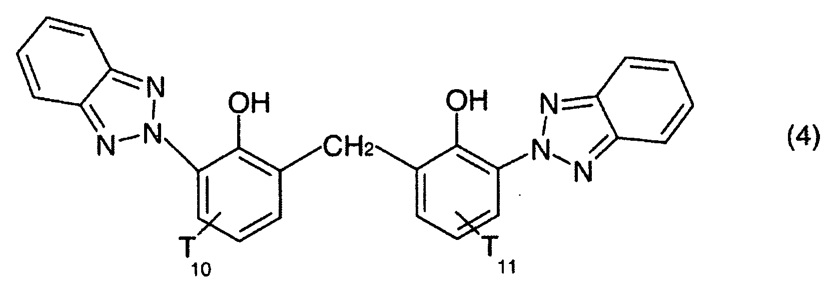 in which the radicals T₁₀ and T₁₁, which may be identical or different, denote a C₁-C₁₈ alkyl radical that may be substituted with one or more radicals chosen from C₁-C₄ alkyl, C₅-C₁₂ cycloalkyl and an aryl residue.

28. Composition according to Claim 27, in which the compound of formula (4) is chosen from the group consisting of the compounds having the following structures:

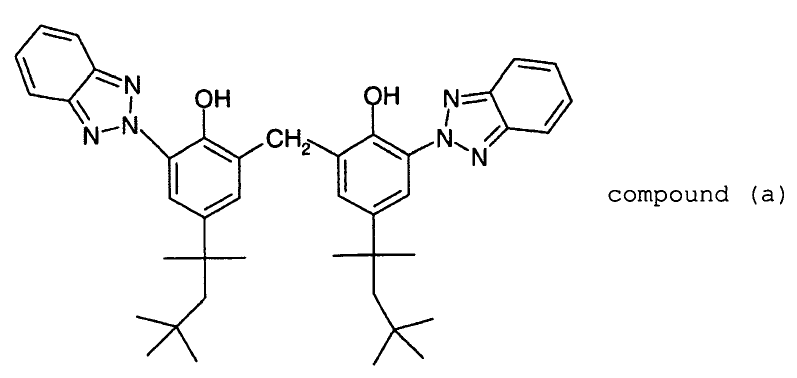

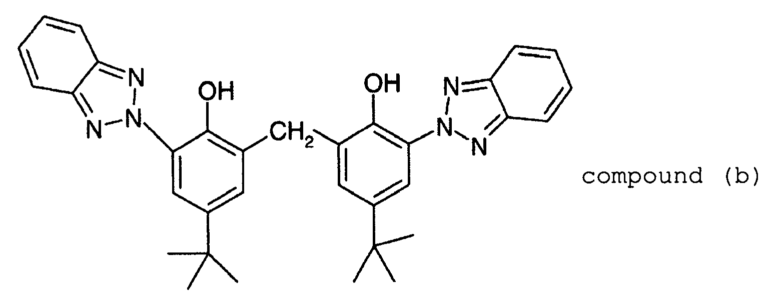

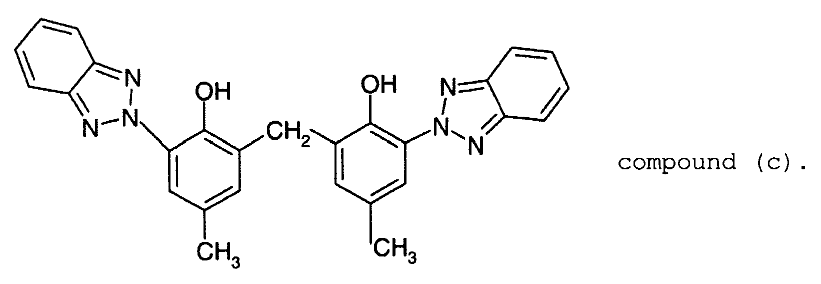

29. Composition according to Claim 16, in which the organic screening agents of the vinyl amide type correspond to the following formula:
T₁₂-(Y)r-C(=O)-C(T₁₃)=C(T₁₄)-N(T₁₅)(T₁₆) (5)
in which T₁₂ is a C₁-C₁₈ and preferably C₁-C₅ alkyl radical or a phenyl group optionally substituted with one, two or three radicals chosen from OH, C₁-C₁₈ alkyl and C₁-C₈ alkoxy, or a group -C(=O)-OT₁₇ in which T₁₇ is a C₁-C₁₈ alkyl; T₁₃, T₁₄, T₁₅ and T₁₆, which may be identical or different, denote a C₁-C₁₈ and preferably C₁-C₅ alkyl radical or a hydrogen atom; Y is N or O and r is 0 or 1.

30. Composition according to Claim 29, in which the compounds of formula (5) are chosen from:
- 4-octylamino-3-penten-2-one;
- ethyl 3-octylamino-2-butenoate;
- 3-octylamino-1-phenyl-2-buten-1-one,
- 3-dodecylamino-1-phenyl-2-buten-1-one.

31. Composition according to Claim 16, in which the organic screening agents of the cinnamamide type correspond to the following formula: 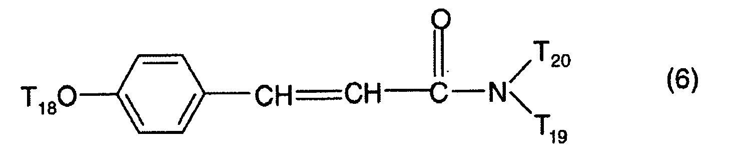 in which OT₁₈ is a hydroxyl or C₁-C₄ alkoxy, preferably methoxy or ethoxy, radical; T₁₉ is hydrogen or C₁-C₄ alkyl, preferably methyl or ethyl; T₂₀ is a group -(CONH)s-phenyl in which s is 0 or 1 and the phenyl group may be substituted with one, two or three groups chosen from OH, C₁-C₁₈ alkyl and C₁-C₈ alkoxy, or a group -C(=O)-OT₂₁ in which T₂₁ is a C₁-C₁₈ alkyl.

32. Composition according to Claim 16, in which the insoluble UV-screening agent is a cinnamamide dimer.

33. Composition according to Claim 32, in which the insoluble UV-screening agent is the compound of structure:

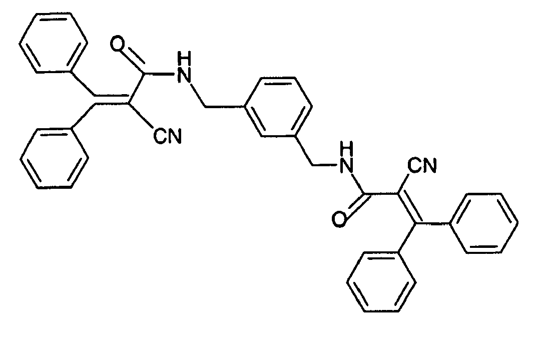

34. Composition according to Claim 16, in which the insoluble UV-screening agents of the benzazole type are chosen from those corresponding to one of the formulae (7), (8) and (9) below: 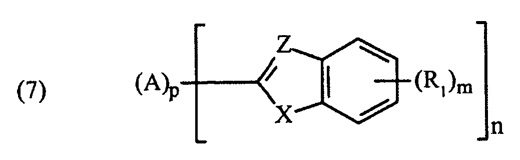 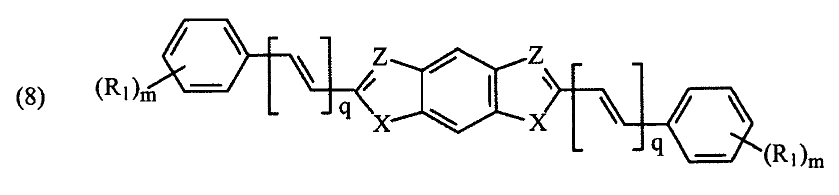 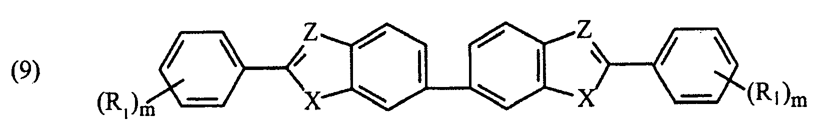 in which
each of the symbols X independently represents an oxygen or sulphur atom or a group NR₂,
each of the symbols Z independently represents a nitrogen atom or a CH group,
each of the symbols R₁ independently represents an OH group, a halogen atom, a linear or branched C₁₋₈ alkyl group optionally containing a silicon atom, or a linear or branched C₁₋₈ alkoxy group,
each of the numbers m is independently 0, 1 or 2,
n represents an integer between 1 and 4 inclusive,
p is equal to 0 or 1,
each of the numbers q is independently equal to 0 or 1,
each of the symbols R₂ independently represents a hydrogen atom or a linear or branched C₁₋₈ alkyl or benzyl group optionally containing a silicon atom,
A represents a radical of valency n chosen from those of formulae: 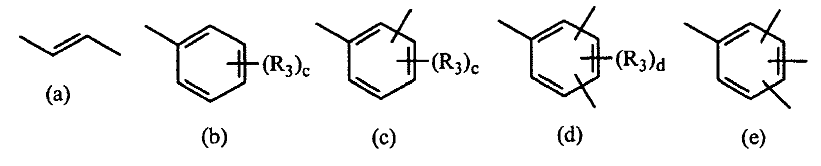 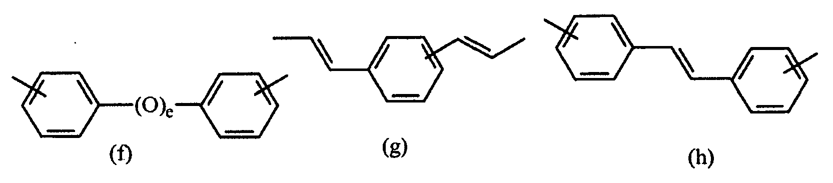 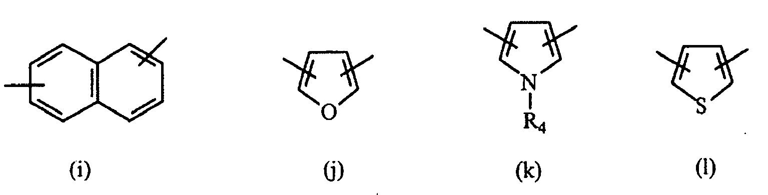 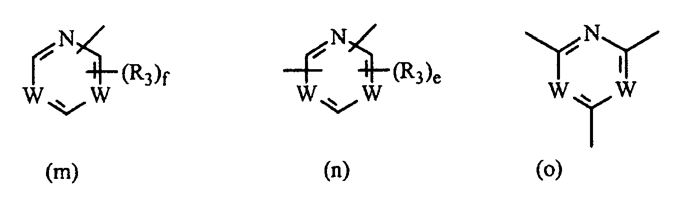 in which
each of the symbols R₃ independently represents a halogen atom or a linear or branched C₁₋₄ alkyl or alkoxy group, or hydroxyl,
R₄ represents a hydrogen atom or a linear or branched C₁₋₄ alkyl group,
c = 0 - 4, d = 0 - 3, e = 0 or 1 and f = 0 - 2.

35. Composition according to Claim 34, in which the benzazole compound of formula (7) is chosen from 2-benzoxazol-2-yl-4-methylphenol, 2-(1H-benzimidazol-2-yl)-4-methoxyphenol or 2-benzothiazol-2-ylphenol, 2,2'-bis(benzimidazole), 5,5',6,6'-tetramethyl-2,2'-bis(benzimidazole), 5,5'-dimethyl-2,2'-bis(benzimidazole), 6-methoxy-2,2'-bis(benzimidazole), 2-(1H-benzimidazol-2-yl)benzothiazole, 2-(1H-benzimidazol-2-yl)benzoxazole and N,N'-dimethyl-2,2'-bis(benzimidazole), 1,4-phenylenebis(2-benzoxazolyl), 1,4-phenylenebis(2-benzimidazolyl), 1,3-phenylenebis(2-benzoxazolyl), 1,2-phenylenebis(2-benzoxazolyl), 1,2-phenylenebis(benzimidazolyl), 1,4-phenylenebis(N-2-ethylhexyl-2-benzimidazolyl) and 1,4-phenylenebis(N-trimethylsilylmethyl-2-benzimidazolyl), 2-(2-benzofuryl)benzoxazole, 2-(benzofuryl)-5-methylbenzoxazole and 2-(3-methyl-2-benzofuryl)benzoxazole.

36. Composition according to Claim 34, in which the benzazole compound of formula (8) is chosen from 2,6-diphenyl-1,7-dihydrobenzo[1,2-d;4,5-d']diimidazole, 2,6-distyryl-1,7-dihydrobenzo[1,2-d;4,5-d']diimidazole and 2,6-di(p-tert-butylstyryl)-1,7-dihydrobenzo[1,2-d;4,5-d']diimidazole.

37. Composition according to Claim 34, in which the benzazole compound of formula (9) is 5,5'-bis[(2-phenyl)benzimidazole].

38. Composition according to either of Claims 34 and 35, in which the insoluble UV-screening agents of the benzazole type are chosen from 2-(1H-benzimidazol-2-yl)benzoxazole, 6-methoxy-2,2'-bis-(benzimidazole), 2-(1H-benzimidazol-2-yl)benzothiazole, 1,4-phenylenebis(2-benzoxazolyl), 1,4-phenylenebis(2-benzimidazolyl), 1,3-phenylenebis(2-benzoxazolyl), 1,2-phenylenebis(2-benzoxazolyl), 1,2-phenylenebis(2-benzimidazolyl) and 1,4-phenylenebis(N-trimethylsilylmethyl-2-benzimidazolyl).

39. Composition according to any one of Claims 1 to 15, in which the insoluble UV-screening agents are multivalent metal salts of sulphonic or carboxylic organic UV-screening agents.

40. Composition according to Claim 39, in which the insoluble UV-screening agents are chosen from the multivalent metal salts of sulphonated benzylidenecamphor derivatives; the multivalent metal salts of sulphonated benzimidazole derivatives; the multivalent metal salts of cinnamic acid derivatives.

41. Composition according to any one of Claims 1 to 15, in which the insoluble UV-screening agents are complexes of multivalent metals or of ammonium or of substituted ammonium of UV-A and/or UV-B organic screening agents.

42. Composition according to any one of Claims 1 to 15, in which the insoluble mineral UV-screening agents are coated or uncoated metal oxide pigments.

43. Composition according to Claim 42, in which the metal oxide pigments have a mean primary particle size of between 5 nm and 100 nm and preferably between 10 nm and 50 nm.

44. Composition according to Claim 43, in which the metal oxide pigments are titanium, iron, zinc, zirconium or cerium oxide pigments.

45. Composition according to Claim 44, in which the metal oxide pigments are pigments of titanium oxide that is amorphous or crystallized in rutile and/or anatase form.

46. Composition according to any one of Claims 1 to 45, **characterized in that** the insoluble UV-screening agent(s) is(are) present in a total concentration of between 0.1% and 25% by weight approximately and preferably between 0.2% and 20% by weight approximately relative to the total weight of the composition.

47. Composition according to any one of Claims 1 to 46, **characterized in that** it also comprises at least one agent for artificially tanning and/or browning the skin.

48. Composition according to any one of Claims 1 to 47, **characterized in that** it also comprises at least one UV-A-active and/or UV-B-active soluble organic UV-screening agent.

49. Composition according to Claim 48, in which the soluble organic UV-screening agents are chosen especially from anthranilates; cinnamic derivatives; dibenzoylmethane derivatives; salicylic derivatives; camphor derivatives; triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; screening polymers and screening silicones; dimers derived from α-alkylstyrene; 4,4-diarylbutadienes, and mixtures thereof.

50. Composition according to Claim 49, in which the soluble organic UV-screening agents are chosen from:
- ethylhexyl salicylate,
- butylmethoxydibenzoylmethane,
- ethylhexyl methoxycinnamate,
- octocrylene,
- phenylbenzimidazolesulphonic acid,
- terephthalylidenedicamphorsulphonic acid,
- benzophenone-3,
- benzophenone-4,
- benzophenone-5,
- 4-methylbenzylidenecamphor,
- benzimidazilate,
- anisotriazine,
- ethylhexyltriazone,
- diethylhexylbutamidotriazone,
- drometrizole trisiloxane,
and mixtures thereof.

51. Composition according to any one of Claims 1 to 50, **characterized in that** it also comprises at least one adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellents, fragrances, preserving agents, surfactants, fillers, photoprotective agents, polymers other than those of the invention, propellants, acidifying or basifying agents and colorants.

52. Composition according to any one of Claims 1 to 51, **characterized in that** it is a composition for protecting the human epidermis or an antisun composition and it is in the form of a nonionic vesicular dispersion, an emulsion, in particular an emulsion of oil-in-water type, a cream, a milk, a gel, a cream-gel, a suspension, a dispersion, a powder, a solid tube, a mousse or a spray.

53. Composition according to any one of Claims 1 to 52, **characterized in that** it is a makeup composition for the eyelashes, the eyebrows or the skin, and it is in solid or pasty, anhydrous or aqueous form or in the form of an emulsion, a suspension or a dispersion.

54. Composition according to any one of Claims 1 to 52, **characterized in that** it is a composition for protecting the hair against ultraviolet rays and it is in the form of a shampoo, a lotion, a gel, an emulsion or a nonionic vesicular dispersion.

55. Use of an amphiphilic polymer comprising at least one ethylenically unsaturated monomer containing a sulphonic group, in free form or partially or totally neutralized form, and comprising at least one hydrophobic portion, as defined in any one of Claims 1 to 15, to manufacture a photoprotective cosmetic or dermatological composition containing at least one insoluble organic UV-screening agent, with the aim of increasing the water resistance of its screening power.

## Patentansprüche

1. Kosmetische oder dermatologische Zusammensetzung, die mindestens ein Lichtschutzsystem enthält, das befähigt ist, UV-Strahlung zu filtern und mindestens ein organisches oder anorganisches, unlösliches UV-Filter mit einer Partikelgröße von 5 nm bis 5 µm enthält, **dadurch gekennzeichnet, dass** sie ferner mindestens ein amphiphiles Polymer enthält, das mindestens ein Monomer mit ethylenisch ungesättigter Bindung und einer Sulfonsäuregruppe in freier Form oder ganz oder teilweise neutralisiert aufweist und mindestens einen hydrophobe Teil enthält und das unter den Copolymeren ausgewählt ist, die aus 2-Acrylamido-2-methylpropansulfonsäureeinheiten (AMPS) der folgenden Formel (II): 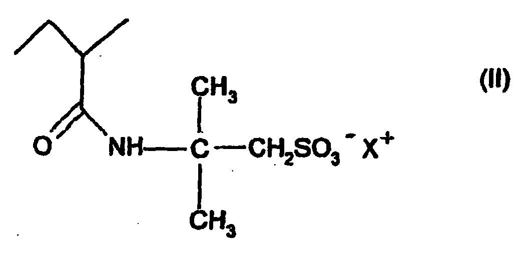 worin X⁺ ein Proton, ein Alkalimetallkation, ein Erdalkalimetallkation oder das Ammoniumion bedeutet,
und Einheiten der folgenden Formel (III) gebildet sind: 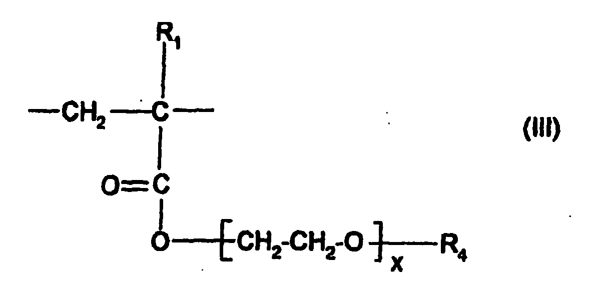 worin x eine ganze Zahl von 3 bis 100, vorzugsweise 5 bis 80 und besonders bevorzugt 7 bis 25 ist; R₁ ein Wasserstoffatom oder eine geradkettige oder verzweigte C₁₋₆-Alkylgruppe bedeutet und R₄ eine geradkettige oder verzweigte Alkylgruppe mit 6 bis 22 Kohlenstoffatomen und noch bevorzugter 10 bis 22 Kohlenstoffatomen ist.

2. Zusammensetzung nach Anspruch 1, **dadurch gekennzeichnet, dass** die amphiphilen Polymere ganz oder teilweise mit einer anorganischen oder organischen Base neutralisiert sind.

3. Zusammensetzung nach Anspruch 1 oder 2, **dadurch gekennzeichnet, dass** die amphiphilen Polymere eine zahlenmittlere Molmasse von 1.000 bis 20.000.000 g/mol aufweisen.

4. Zusammensetzung nach Anspruch 3, **dadurch gekennzeichnet, dass** die zahlenmittlere Molmasse im Bereich von 20.000 bis 5.000.000 g/mol liegt.

5. Zusammensetzung nach Anspruch 4, **dadurch gekennzeichnet, dass** die zahlenmittlere Molmasse im Bereich von 100.000 bis 1.500.000 g/mol liegt.

6. Zusammensetzung nach einem der vorhergehenden Ansprüche, **dadurch gekennzeichnet, dass** die amphiphilen Polymere durch radikalische Polymerisation unter Präzipitation in *tert-*Butanol hergestellt werden.

7. Zusammensetzung nach einem der vorhergehenden Ansprüche, **dadurch gekennzeichnet, dass** die amphiphilen Polymere vernetzt oder nichtvernetzt sind.

8. Zusammensetzung nach Anspruch 7, **dadurch gekennzeichnet, dass** die amphiphilen Polymere vernetzt sind.

9. Zusammensetzung nach Anspruch 8, **dadurch gekennzeichnet, dass** das oder die Vernetzungsmittel unter den Verbindungen mit mehreren olefinischen Doppelbindungen ausgewählt sind.

10. Zusammensetzung nach Anspruch 9, **dadurch gekennzeichnet, dass** das oder die Vernetzungsmittel unter Methylen-bisacrylamid, Allylmethacrylat oder Trimethylolpropantriacrylat (TMPTA) ausgewählt sind.

11. Zusammensetzung nach einem der Ansprüche 8 bis 10, **dadurch gekennzeichnet, dass** der Vernetzungsgrad vorzugsweise im Bereich von 0,01 bis 10 Mol-% und insbesondere 0,2 bis 2 Mol-%, bezogen auf das Polymer, liegt.

12. Zusammensetzung nach einem der vorhergehenden Ansprüche, **dadurch gekennzeichnet, dass** x = 25, R₁ Methyl bedeutet und R₄ *n*-Dodecyl ist.

13. Zusammensetzung nach einem der vorhergehenden Ansprüche, **dadurch gekennzeichnet, dass** der prozentuale molare Anteil der Einheiten der Formel (III) in den Polymeren im Bereich von 50,1 bis 99,9 % liegt.

14. Zusammensetzung nach einem der vorhergehenden Ansprüche, **dadurch gekennzeichnet, dass** der prozentuale molare Anteil der Einheiten der Formel (III) in den Polymeren im Bereich von 0,1 bis 50 % liegt.

15. Zusammensetzung nach einem der vorhergehenden Ansprüche, **dadurch gekennzeichnet, dass** die amphiphilen Polymere in Konzentrationen von 0,01 bis 30 Gew.-%, vorzugsweise 0,1 bis 10 %, noch bevorzugter 0,1 bis 5 Gew.-% und besonders bevorzugt 0,5 bis 2 Gew.-%, bezogen auf das Gesamtgewicht der Zusammensetzung, vorliegen.

16. Zusammensetzung nach einem der Ansprüche 1 bis 15, wobei der oder die unlöslichen organischen UV-Filter unter den organischen UV-Filtern vom Oxanilidtyp, Triazintyp, Triazoltyp, Vinylamidtyp, Cinnamidtyp oder Benzazoltyp ausgewählt sind

17. Zusammensetzung nach Anspruch 16, wobei die UV-Filter vom Oxanilidtyp die folgende Formel aufweisen: 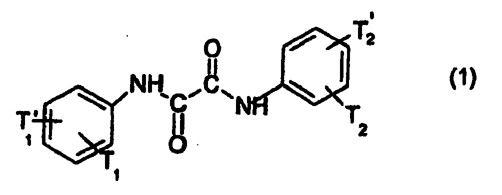 worin T₁, T'₁, T₂ und T'₂, die identisch oder voneinander verschieden sind, eine C₁₋₈-Alkylgruppe oder eine C₁₋₈-Alkoxygruppe bedeuten.

18. Zusammensetzung nach Anspruch 17, wobei die UV-Filter vom Oxanilidtyp unter den folgenden Verbindungen ausgewählt sind:

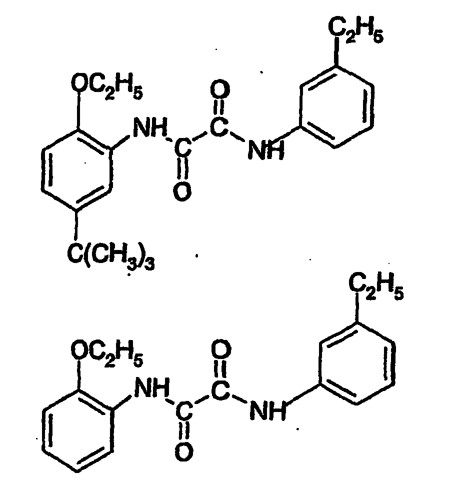

19. Zusammensetzung nach Anspruch 16, wobei die UV-Filter vom Triazintyp unter den unlöslichen s-Triazinderivaten ausgewählt sind, die Benzalmalonatgruppen und/oder Phenylcyanoacrylatgruppen aufweisen.

20. Zusammensetzung nach Anspruch 19, wobei die UV-Filter vom Triazintyp unter den folgenden Verbindungen ausgewählt sind:
- 2,4,6-Tris(diethyl-4'-aminobenzalmalonat)-s-triazin,
- 2,4,6-Tris(düsopropyl-4'-aminobenzalmalonat)-s-triazin,
- 2,4,6-Tris(dimethyl-4'-aminobenzalmalonat)-s-triazin,
- 2,4,6-Tris(ethyl-α-cyano-4-aminocinnamat)-s-triazin.

21. Zusammensetzung nach Anspruch 16, wobei die unlöslichen UV-Filter vom Triazintyp ausgewählt sind unter: 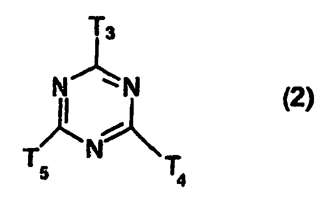 worin die Gruppen T₃, T₄ und T₅ unabhängig voneinander Phenyl, Phenoxy oder Pyrrolo bedeuten, wobei die Phenyl-, Phenoxy- oder Pyrrologruppen gegebenenfalls mit einem, zwei oder drei Substituenten substituiert sind, die ausgewählt sind unter: OH, C₁₋₁₈-Alkyl oder C₁₋₁₈-Alkoxy, C₁₋₁₈-Carboxyalkyl, C₅₋₈-Cycloalkyl, Methylidencampher, -(CH=CH)ₙ(CO)-OT₆, wobei T₆ entweder C₁₋₁₈-Alkyl oder Cinnamyl bedeutet und n 0 oder 1 ist.

22. Zusammensetzung nach Anspruch 16, wobei die UV-Filter vom Triazintyp unter den unlöslichen s-Triazinderivaten ausgewählt sind, die Benzotriazolgruppen und/oder Benzothiazolgruppen aufweisen.

23. Zusammensetzung nach Anspruch 22, wobei die UV-Filter vom Triazintyp ausgewählt sind unter:
- 2,4,6-Tris[(3'-benzotriazol-2-yl-2'-hydroxy-5'-methyl)-phenylamino]-s-triazin,
- 2,4,6-Tris[(3'-benzotriazol-2-yl-2'-hydroxy-5'-t-octyl)-phenylamino]-s-triazin.

24. Zusammensetzung nach Anspruch 16, wobei die organischen UV-Filter vom Triazoltyp der folgenden Formel (3) entsprechen: 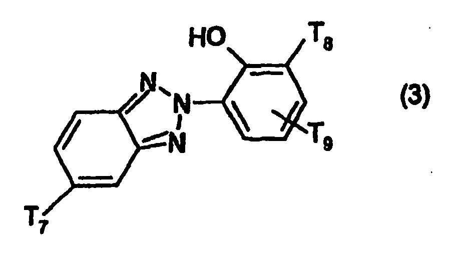 wobei T₇ ein Wasserstoffatom oder eine C₁₋₁₈-Alkylgruppe bedeutet und T₈ und T₉, die identisch oder voneinander verschieden sind, eine gegebenenfalls mit einer Phenylgruppe substituierte C₁₋₁₈-Alkylgruppe bedeuten.

25. Zusammensetzung nach Anspruch 24, wobei die Verbindung der Formel (3) unter den folgenden Verbindungen ausgewählt ist.

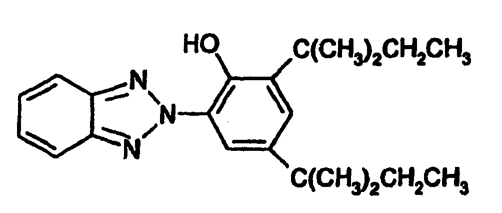

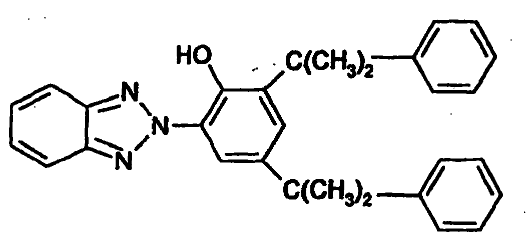

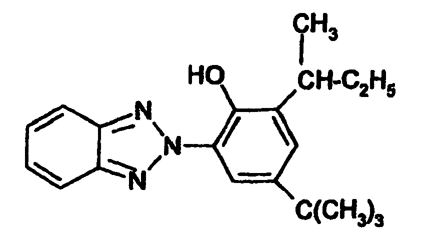

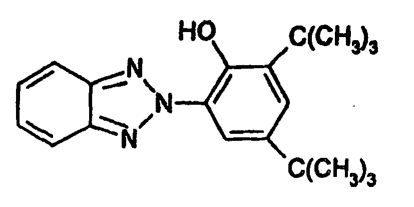

26. Zusammensetzung nach Anspruch 16, wobei das unlösliche UV-Filter das [2,4'-Dihydroxy-3-(2H-benzotriazol-2-yl)-5-(1,1,3,3-tetramethylbutyl)-2'-n-octoxy-5'-benzoyl]diphenylmethan der folgenden Struktur ist:

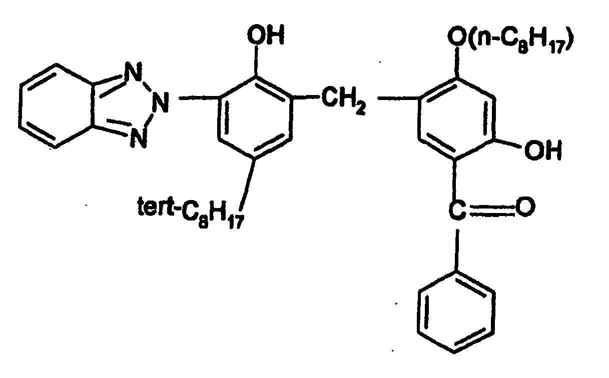

27. Zusammensetzung nach Anspruch 16, wobei die organischen UV-Filter vom Triazoltyp unter den Methylen-bis-(hydroxyphenylbenzotriazol)-derivaten der folgenden Struktur ausgewählt sind: 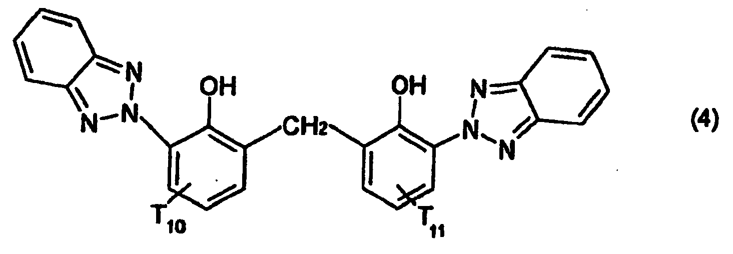 worin die Gruppen Tio und T₁₁, die identisch oder voneinander verschieden sind, eine C₁₋₈-Alkylgruppe, die mit einer oder mehreren Gruppen substituiert sein kann, die unter den C₁₋₄-Alkylgruppen oder C₅₋₁₂-Cycloakylgruppen ausgewählt sind, oder eine Arylgruppe bedeuten.

28. Zusammensetzung nach Anspruch 27, wobei die Verbindung der Formel (4) unter den Verbindungen der folgenden Strukturen ausgewählt ist:

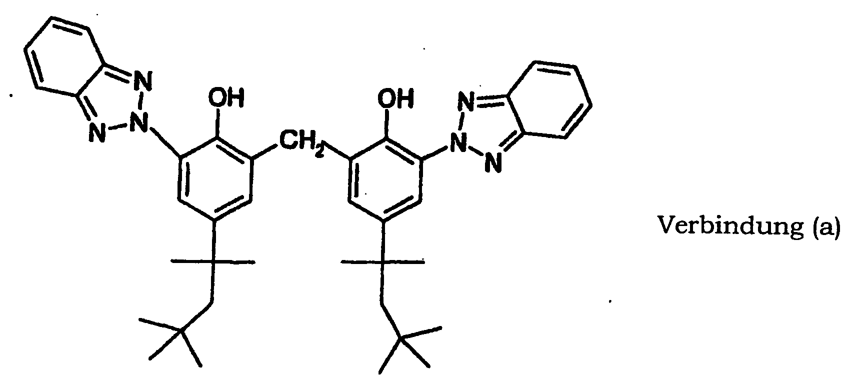

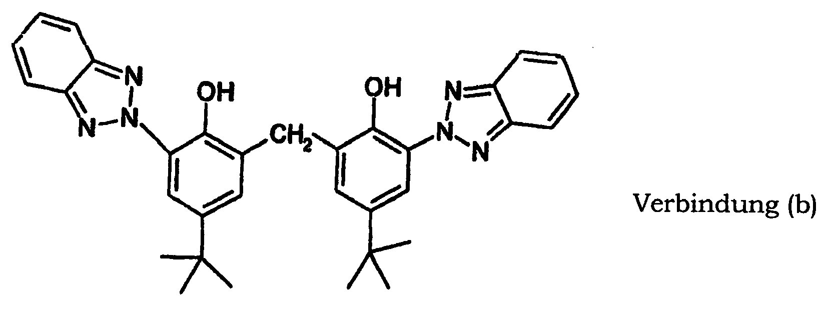

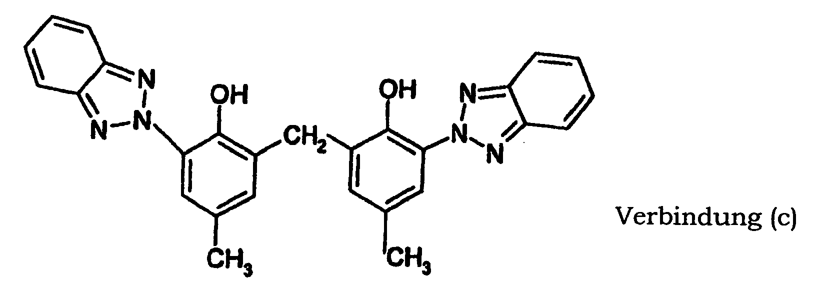

29. Zusammensetzung nach Anspruch 16, wobei die organischen Filter vom Vinylamidtyp der folgenden Formel entsprechen:
**T₁₂-(Y)r-C(=O)-C(T₁₃)=C(T₁₄)-N(T₁₅)(T₁₆)** **(5)**
worin T₁₂ eine C₁₋₁₈-Alkylgruppe und vorzugsweise eine C₁₋₅-Alkylgruppe oder eine Phenylgruppe, die gegebenenfalls mit einer, zwei oder drei Gruppen substituiert ist, die ausgewählt sind unter: OH, C₁₋₁₈-Alkyl oder C₁₋₈-Alkoxy, oder -C(=O)-OT₁₇ bedeutet, wobei T₁₇ eine C₁₋₁₈-Alkylgruppe ist; die Gruppen T₁₃, T₁₄, T₁₅ und T₁₆, die identisch oder voneinander verschieden sind, eine C₁₋₁₈-Alkylgruppe und vorzugsweise eine C₁₋₅-Alkylgruppe oder ein Wasserstoffatom bedeuten; Y N oder O ist und r 0 oder 1 bedeutet.

30. Zusammensetzung nach Anspruch 29, wobei die Verbindungen der Formel (5) ausgewählt sind unter:
- 4-Octylamino-3-penten-2-on;
- Ethyl-3-octylamino-2-butenoat;
- 3-Octylamino-1-phenyl-2-buten-1-on und
- 3-Dodecylamino-1-phenyl-2-buten-1-on.

31. Zusammensetzung nach Anspruch 16, wobei die organischen Filter vom Cinnamamidtyp der folgenden Formel entsprechen: 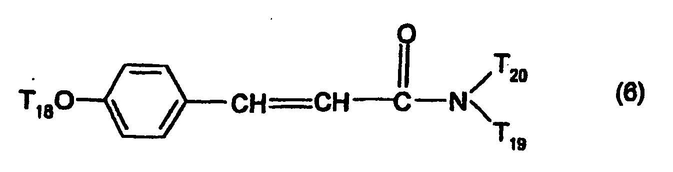 worin OT₁₈ eine Hydroxygruppe oder eine C₁₋₄-Alkoxygruppe ist, vorzugsweise Methoxy oder Ethoxy; T₁₉ Wasserstoff, C₁₋₄-Alkyl und vorzugsweise Methyl oder Ethyl bedeutet; T₂₀ eine Gruppe (CONH)ₛ-phenyl, wobei s 0 oder 1 bedeutet und die Phenylgruppe mit einer, zwei oder drei Gruppen substituiert sein kann, die unter OH, C₁₋₁₈-Mkyl oder C₁₋₈-Alkoxy ausgewählt sind, oder -C(=O)-OT₂₁ bedeutet, wobei T₂₁ eine C₁₋₁₈-Alkylgruppe ist.

32. Zusammensetzung nach Anspruch 16, wobei das unlösliche UV-Filter ein Cinnamamid-Dimer ist.

33. Zusammensetzung nach Anspruch 32, wobei das unlösliche UV-Filter die Verbindung der folgenden Struktur ist:

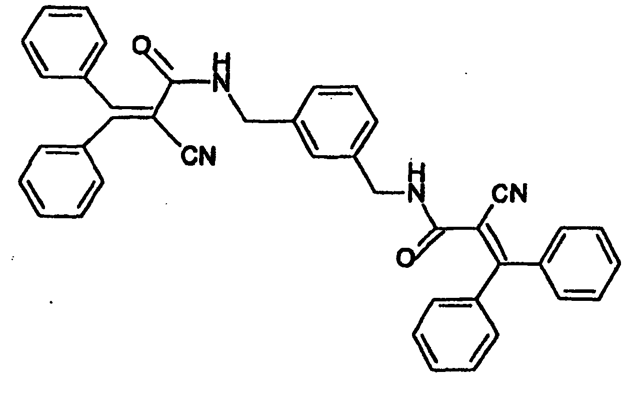

34. Zusammensetzung nach Anspruch 16, wobei die unlöslichen Filter vom Benzazoltyp unter den Verbindungen ausgewählt sind, die einer der folgenden Formeln (7), (8) und (9) entsprechen: 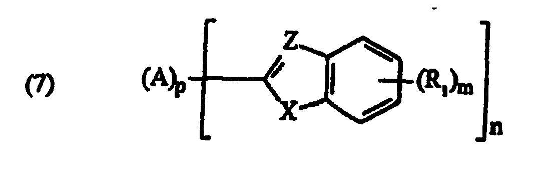 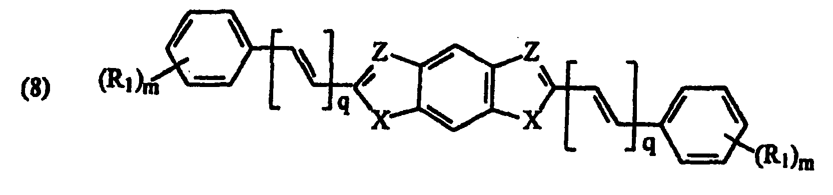 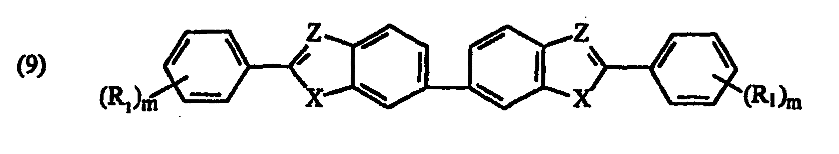 worin bedeuten:
jedes der Symbole X unabhängig ein Sauerstoffatom oder eine Schwefelatom oder eine Gruppe NR₂,
jedes der Symbole Z unabhängig ein Stickstoffatom oder eine Gruppe CH,
jedes der Symbole R₁ unabhängig eine Gruppe OH, ein Halogenatom, eine geradkettige oder verzweigte C₁₋₈-Alkylgruppe, die gegebenenfalls ein Siliciumatom enthält, oder eine geradkettige oder verzweigte C₁₋₈-Alkoxygruppe,
jede der Zahlen m 0, 1 oder 2,
n eine ganze Zahl von 1 bis 4,
p 0 oder 1,
jede der Zahlen q unabhängig 0 oder 1,
jedes der Symbole R₂ unabhängig ein Wasserstoffatom, eine Benzylgruppe oder eine geradkettige oder verzweigte C₁₋₈-Alkylgruppe, die gegebenenfalls ein Siliciumatom enthält,
A eine Gruppe der Valenz n, die unter den Gruppen der folgenden Formel ausgewählt ist: 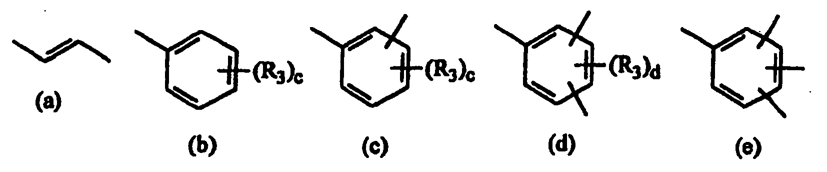 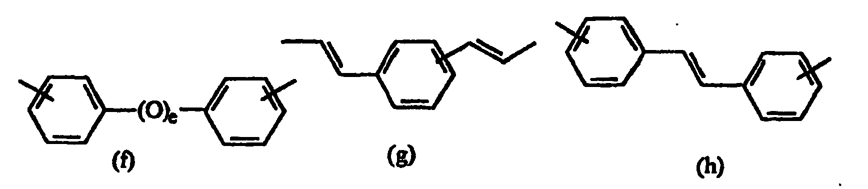 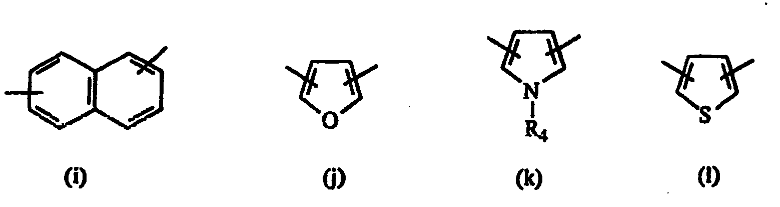 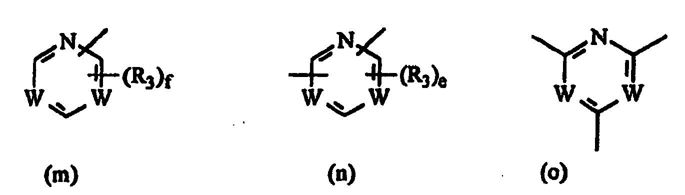
worin bedeuten:
jedes der Symbole R₃ unabhängig ein Halogenatom, eine geradkettige oder verzweigte Alkyl- oder Alkoxygruppe mit 1 bis 4 Kohlenstoffatomen oder Hydroxy,
R₄ ein Wasserstoffatom oder eine geradkettige oder verzweigte C₁₋₄-Alkylgruppe, c = 0-4, d = 0-3, e = 0 oder 1 und f = 0-2.

35. Zusammensetzung nach Anspruch 34, wobei das Benzazol der Formel (7) unter 2-Benzoxazol-2-yl-4-methylphenol, 2-(1H-benzimidazol-2-yl)-4-methoxyphenol oder 2-Benzothiazol-2-ylphenol, 2,2'-Bis-benzimidazol, 5,5'-6,6'-Tetramethyl-2,2'-bis-benzimidazol, 5,5'-Dimethyl-2,2'-bis-benzimidazol, 6-Methoxy-2,2'-bis-benzimidazol, 2-(1H-benzimidazol-2-yl)-benzothiazol, 2-(1H-benzimidazol-2-yl)-benzoxazol und N,N'-Dimethyl-2,2'-bis-benzimidazol, 1,4-Phenylen-bis-(2-benzoxazolyl), 1,4-Phenylen-bis-(2-benzimidazolyl), 1,3-Phenylen-bis-(2-benzoxazolyl), 1,2-Phenylen-bis-(2-benzoxazolyl), 1,2-Phenylen-bis-(benzimidazolyl), 1,4-Phenylen-bis-(N-2-ethylhexyl-2-benzimidazolyl), 1,4-Phenylen-bis-(N-trimethylsilylmethyl-2-benzimidazolyl), 2-(2-Benzofuranyl)-benzoxazol, 2-(Benzofuranyl)-5-methylbenzoxazol, und 2-(3-Methyl-2-benzofuranyl)-benzoxazol ausgewählt ist.

36. Zusammensetzung nach Anspruch 34, wobei das Benzazol der Formel (8) unter 2,6-Diphenyl-1,7-dihydro-benzo[1,2-d;4,5-d']-di-imidazol, 2,6-Distyryl-1,7-dihydro-benzo[1,2-d;4,5-d']-di-imidazol und 2,6-Di-(p-*tert* butylstyryl)-1,7-dihydro-benzo[1,2-d;4,5-d']-di-imidazol ausgewählt ist.

37. Zusammensetzung nach Anspruch 34, wobei das Benzazol der Formel (9) das 5,5'-Bis-[(2-phenyl)-benzimidazol] ist.

38. Zusammensetzung nach einem der Ansprüche 34 und 35, wobei die unlöslichen UV-Filter vom Benzazoltyp ausgewählt sind unter: 2-(1H-benzimidazol-2-yl)-benzoxazol, 6-Methoxy-2,2'-bis-benzimidazol, 2-(1H-benzimidazol-2-yl)-benzothiazol, 1,4-Phenylen-bis-(2-benzoxazolyl), 1,4-Phenylen-bis-(2-benzimidazolyl), 1,3-Phenylen-bis-(2-benzoxazolyl), 1,2-Phenylen-bis-(2-benzoxazolyl), 1,2-Phenylen-bis-(2-benzimidazolyl) und 1,4-Phenylen-bis-(N-trimethylsilylmethyl-2-benzimidazolyl).

39. Zusammensetzung nach einem der Ansprüche 1 bis 15, wobei die unlöslichen UV-Filter Salze mehrwertiger Metalle von organischen, im UV-Bereich filternden Sulfon- oder Carbonsäuren sind.

40. Zusammensetzung nach Anspruch 39, wobei die unlöslichen UV-Filter unter den Salzen mehrwertiger Metalle von Benzylidencamphersulfonsäurederivaten; Salzen mehrwertiger Metalle von Benzimidazolsulfonsäurederivaten; und Salzen mehrwertiger Metalle von Zimtsäure ausgewählt sind.

41. Zusammensetzung nach einem der Ansprüche 1 bis 15, wobei die unlöslichen UV-Filter Komplexe von mehrwertigen Metallen oder Ammonium oder substituiertem Ammonium und organischen UV-A- und/oder UV-B-Filtern sind.

42. Zusammensetzung nach einem der Ansprüche 1 bis 15, wobei die anorganischen unlöslichen UV-Filter Pigmente von Metalloxiden sind, die umhüllt oder nicht umhüllt sind.

43. Zusammensetzung nach Anspruch 42, wobei die Pigmente von Metalloxiden eine mittlere Größe der Primärteilchen im Bereich von 5 bis 100 nm und vorzugsweise 10 bis 50 nm aufweisen.

44. Zusammensetzung nach Anspruch 43, wobei es sich bei den Pigmenten von Metalloxiden um Pigmente von Titanoxid, Eisenoxid, Zinkoxid, Zirconiumoxid oder Ceroxid handelt.

45. Zusammensetzung nach Anspruch 44, wobei es sich bei den Pigmenten von Metalloxiden um Pigmente von amorphem oder in Form von Rutil und/oder Anatas kristallisiertem Titanoxid handelt.

46. Zusammensetzung nach einem der Ansprüche 1 bis 45, **dadurch gekennzeichnet, dass** das oder die unlösliche(n) UV-Filter in einer Gesamtkonzentration von etwa 0,1 bis 25 Gew.-% und vorzugsweise etwa 0,2 bis 20 Gew.-%, bezogen auf das Gesamtgewicht der Zusammensetzung, vorliegen.

47. Zusammensetzung nach einem der Ansprüche 1 bis 46, **dadurch gekennzeichnet, dass** sie ferner mindestens ein Bräunungsmittel und/oder Mittel zur künstlichen Braunfärbung der Haut enthält.

48. Zusammensetzung nach einem der Ansprüche 1 bis 47, **dadurch gekennzeichnet, dass** sie ferner auch mindestens ein im UV-A- und/oder UV-B-Bereich wirksames, lösliches organisches UV-Filter enthält.

49. Zusammensetzung nach Anspruch 48, wobei die löslichen organischen Filter ausgewählt sind unter: Anthranilaten; Zimtsäurederivaten; Dibenzoylmethanderivaten; Salicylsäurederivaten; Campherderivaten; Triazinderivaten; Benzophenonderivaten; β,β-Diphenylacrylatderivaten; Benzotriazolderivaten; Benzalmalonatderivaten, Benzimidazolderivaten; Imidazolinen; Bisbenzoazolylderivaten; Derivaten von *p*-Aminobenzoesäure (PABA); polymeren Filtern und Siliconfiltern; von α-Alkylstyrol abgeleiteten Dimeren; 4,4-Diarylbutadienen und deren Gemischen.

50. Zusammensetzung nach Anspruch 49, wobei die löslichen organischen UV-Filter ausgewählt sind unter:
· Ethylhexyl Salicylate,
· Butyl Methoxydibenzoylmethane,
· Ethylhexyl Methoxycinnamate,
· Octocrylene,
· Phenylbenzimidazole Sulfonic Acid,
· Terephthalylidene Dicamphor Sulfonic Acid,
· Benzophenone-3,
· Benzophenone-4
· Benzophenone-5,
· 4-Methylbenzylidene Camphor,
· Benzimidazilate,
· Anisotriazine,
· Ethylhexyl Triazone,
· Diethylhexyl Butamido Triazone,
· Drometrizole Trisiloxane,
· und deren Gemischen.

51. Zusammensetzung nach einem der Ansprüche 1 bis 50, **dadurch gekennzeichnet, dass** sie ferner mindestens einen Zusatzstoff enthält, der unter den Fettsubstanzen, organischen Lösungsmitteln, ionischen oder nichtionischen Verdickungsmitteln, beruhigenden Stoffen, Antioxidantien, Radikalfängern für freie Radikale, Trübungsmitteln, Stabilisatoren, Emollientien, Siliconen, α-Hydroxysäuren, Schaumverhütungsmitteln, Hydratisierungsmitteln, Vitaminen, insektenabwehrenden Stoffen, Parfums, Konservierungsmitteln, grenzflächenaktiven Stoffen, Füllstoffen, Lichtschutzmitteln, von den erfindungsgemäßen Verbindungen verschiedenen Polymeren, Treibmitteln, Alkalisierungsmitteln oder Ansäuerungsmitteln und Farbmitteln ausgewählt ist.

52. Zusammensetzung nach einem der Ansprüche 1 bis 51, **dadurch gekennzeichnet, dass** es sich um eine Zusammensetzung zum Schutz der menschlichen Epidermis oder ein Sonnenschutzmittel handelt, und **dadurch**, dass sie in Form einer nichtionischen Vesikeldispersion, einer Emulsion, insbesondere einer Emulsion vom Öl-in-Wasser-Typ, als Creme, als Milch, als Gel, als Gelcreme, in Form einer Suspension, als Dispersion, als Puder, als fester Stift, als Schaum oder als Spray vorliegt.

53. Zusammensetzung nach einem der Ansprüche 1 bis 52, **dadurch gekennzeichnet, dass** es sich um eine Zusammensetzung zum Schminken der Wimpern, Augenbrauen oder der Haut handelt, und **dadurch**, dass sie in fester oder pastöser, wasserfreier oder wässriger Form, als Emulsion, Suspension oder Dispersion vorliegt.

54. Zusammensetzung nach einem der Ansprüche 1 bis 52, **dadurch gekennzeichnet, dass** es sich um eine Zusammensetzung zum Schutz der Haare gegen UV-Strahlung handelt, und **dadurch**, dass sie als Haarwaschmittel, Lotion, Gel, Emulsion oder nichtionische Vesikeldispersion vorliegt.

55. Verwendung eines amphiphilen Polymers, das mindestens ein Monomer mit ethylenisch ungesättigter Bindung und einer Sulfonsäuregruppe in freier Form oder ganz oder teilweise neutralisiert aufweist und mindestens einen hydrophobe Teil enthält, wie es in einem der Ansprüche 1 bis 15 definiert ist, zur Herstellung einer kosmetischen oder dermatologischen Zusammensetzung zum Lichtschutz, die mindestens ein unlösliches, organisches UV-Filter enthält, um im Hinblick auf das Filtervermögen die Wasserbeständigkeit (Remanenz gegenüber Wasser) zu erhöhen.
